# EUROPÄISCHE PATENTSCHRIFT

(11) **EP 3 500 553 B1**
(45) Veröffentlichungstag und Bekanntmachung des Hinweises auf die Patenterteilung: **12.05.2021**
(21) Anmeldenummer: 17754336.0
(22) Anmeldetag: 15.08.2017
(51) Int. Cl.: C07C 263/10, C07C 265/14, C07C 209/78, C07C 209/54, C07C 211/50, B01J 19/00

(54) **VERFAHREN ZUR HERSTELLUNG EINES ISOCYANATS UND MINDESTENS EINES WEITEREN CHEMISCHEN PRODUKTS IN EINEM PRODUKTIONSVERBUND**
METHOD AND DEVICE FOR THE PRODUCTION OF VARIOUS CHEMICAL PRODUCTS
PROCEDE ET DISPOSITIF DESTINE A FABRIQUER DIFFERENTS PRODUITS CHIMIQUES

(30) Priorität: 17.08.2016 EP 16184618; 30.06.2017 EP 17178902
(43) Veröffentlichungstag der Anmeldung: 26.06.2019
(73) Patentinhaber: Covestro Intellectual Property GmbH & Co. KG, 51373 Leverkusen (DE)
(72) Erfinder: SPRIEWALD, Jürgen, 25337 Kölln-Reisiek (DE); ADAMSON, Richard, 42799 Leichlingen (DE); KNAUF, Thomas, 41542 Dormagen (DE)
(74) Vertreter: Levpat
(86) Internationale Anmeldenummer: PCT/EP2017/070675
(87) Internationale Veröffentlichungsnummer: WO 2018/033536

(56) Entgegenhaltungen:
- EP-A1- 2 096 102
- EP-A2- 1 854 783
- WO-A1-2004/056761
- WO-A1-2008/148608
- WO-A1-2013/083230
- DE-A1-102013 205 492

## Beschreibung

Die vorliegende Erfindung betrifft ein Verfahren zur Herstellung von Isocyanaten in Verfahrensketten, in denen jeweils ein Isocyanat als Endprodukt über mindestens ein Zwischenprodukt hergestellt wird, wobei die in einer ersten Produktionsanlage in einem Wärme abgebenden Vorgang der Herstellung eines Zwischenprodukts, eines Isocyanat-Endprodukts oder eines für einen Teilschritt der Verfahrenskette erforderlichen Katalysators (*erstes chemisches Produkt*) freiwerdende Wärmeenergie mindestens teilweise zur Erzeugung von Dampf, insbesondere von Wasserdampf, eines Drucks von 1,31 bar_{(abs}.₎ bis 1,91 bar_{(abs}.₎ und einer Temperatur von 107 °C bis 119 °C genutzt wird und der so erzeugte Dampf für die Durchführung eines Wärme verbrauchenden Vorgangs in der Herstellung eines anderen chemischen Produkts (*zweites chemisches Produkt*) in einer zweiten Produktionsanlage eingesetzt wird.

Großtechnische chemische Verfahren zur Erzeugung von Isocyanaten sowie den zu ihrer Herstellung erforderlichen Vorprodukten stehen zur Verfügung. Die Verfahren können diskontinuierlich, semi-kontinuierlich, kontinuierlich oder in Kombination einer dieser drei Varianten betrieben werden. Die Verfahren sind endotherm oder exotherm und können isotherm oder adiabatisch durchgeführt werden. Die Durchführung des Verfahrens kann je nach Art des herzustellenden Isocyanats in der Gasphase oder in der Flüssigphase mit und ohne Lösungsmittel oder in Schmelze erfolgen. Die Aufarbeitung und Reinigung des so erhaltenen Isocyanats kann nach einem der gängigen Verfahren der Technik, wie z. B. Kristallisation, Wäsche, Destillation oder in einer Kombination dieser Aufarbeitungsweisen erfolgen.

Die Güte eines Verfahrens zur Herstellung eines Isocyanats ist einerseits definiert durch den Gehalt an unerwünschten Nebenprodukten im Produkt des Verfahrens. Andererseits ist die Güte eines Verfahrens dadurch definiert, dass der gesamte Prozess von Inbetriebnahme, Produktion im Sollzustand bis zur Außerbetriebnahme des Prozesses ohne technischen Produktionsausfall und ohne Probleme, die zu einem Eingriff in den Prozess nötigen würden, betrieben werden kann, und dass es nicht zu Verlusten an Einsatzstoffen, Zwischenprodukten oder an Endprodukt kommt.

Idealerweise sind daher die großtechnischen Anlagen zur Durchführung solcher Herstellungsverfahren derart ausgelegt, dass bei einer passenden Qualität der eingesetzten Hilfs- und Einsatzstoffe und richtiger Wahl der Verfahrensparameter wie Druck, Temperatur, Mengenverhältnisse und Konzentrationen der Hilfs- und Einsatzstoffe etc. die Verfahren robust verlaufen. Das bedeutet, dass es in solchen kontinuierlich betriebenen Großanlagen idealerweise nicht zu Problemen wie etwa der Entstehung von Niederschlägen kommt, die sich am Anlagenequipment absetzen oder Rohrleitungen verstopfen können.

Um die Wirtschaftlichkeit weiter zu steigern, wurde schon früh die Effizienz des Energieverbrauches solcher großtechnischer Anlagen gesteigert. Durch sinnvolle Wärmeintegration zwischen den einzelnen Anlagenteilen einer Produktionsanlage kann der Energieverbrauch stark reduziert werden. Es gilt, die sinnvolle Nutzung von überschüssigen Energien, zum Beispiel aus einem adiabatischen Prozess, so weit wie möglich technisch zu realisieren.

So befasst sich z. B. WO 2004/056 761 A1 mit der Wärmeintegration in einem Verfahren zur Herstellung von Isocyanaten durch Umsetzung von Aminen mit Phosgen im Beisein von inerten organischen Lösungsmitteln in einem Reaktor und anschließender Aufarbeitung des den Reaktor verlassenden Reaktionsgemisches. Die Wärmeintegration wird derart realisiert, dass die Lösungsmittelabtrennung in einem zwei- oder mehrstufigen, vorzugsweise zweistufigen destillativen Verfahren erfolgt und das Lösungsmittel in einem ersten Apparat, vorzugsweise einer Destillationskolonne, bei 0,1 bis 15 bar, bevorzugt 0,5 bis 3 bar, und in einem zweiten oder weiteren Apparaten, vorzugsweise ebenfalls Destillationskolonnen, bei 1 bis 900 mbar, bevorzugt 50 bis 500 mbar, abgetrennt wird, wobei die Kondensationswärme des Lösungsmitteldampfes, auch als Brüden bezeichnet, aus dem ersten Apparat zur partiellen oder vollständigen Verdampfung von Lösungsmittel im zweiten Apparat verwendet wird.

In der ersten Kolonne wird Lösungsmitteldampf destillativ abgetrennt. Der flüssige Anteil, der als Sumpfaustrag dieser Kolonne anfällt, wird vor oder im zweiten Apparat auf das niedrigere Druckniveau des zweiten Apparates entspannt und dem zweiten Apparat zugeführt, in welchem das restliche Lösungsmittel abgeführt wird. Die Übertragung der Energie der Brüden aus dem ersten Apparat auf die Flüssigphase des zweiten kann insbesondere unter Verwendung eines Wärmeaustauschers, beispielsweise eines Kreuzstromapparates, erfolgen, in dem die kondensierenden Brüden die Verdampfung der flüssigen Phase aus dem zweiten Apparat der zweiten Kolonne bewirken. Dabei können die Brüden und Flüssigphase im Gleich- oder im Gegenstrom geführt werden. Die Brüden können im Mantel- oder im Produktraum des Wärmeaustauschers kondensiert werden. Die Flüssigkeit der zweiten Kolonne kann aus dem Sumpf, von einem Boden, einem Flüssigkeitssammler oder aus dem Zulauf entnommen werden. Es wird keine weitere Verwendung von den nach der internen Wärmeintegration verbliebenen Energien beschrieben.

Auch die EP 1 854 783 A2 befasst sich mit der Herstellung von Isocyanaten. In dieser Anmeldung wird ebenfalls eine Wärmeintegration bei der Lösungsmittelrückgewinnung beschrieben. In einer bevorzugten Ausführungsform des Verfahrens wird der in Schritt d) erhaltene und in Schritt e) zumindest teilweise zurückgeführte Lösungsmittel enthaltende Strom in einer speziellen Destillationsstufe von Restmengen an Phosgen befreit. Besonders bevorzugt wird bei dieser destillativen Abtrennung der Restmengen an Phosgen die fühlbare Wärme des wiedergewonnenen Lösungsmittelstroms ganz oder teilweise als Energiequelle für diesen Abtrennungsschritt verwendet. Dies kann beispielsweise dadurch erfolgen, dass der Zulauf in die Destillationskolonne über einen Wärmeaustauscher den Kolonnensumpf beheizt. Da normalerweise das destillativ abgetrennte Lösungsmittel bei einer Temperatur von > 100 °C anfällt, das zur Erzeugung der Lösung von Amin in dem Lösungsmittel dagegen für optimale Phosgenierbedingungen < 50 °C haben sollte, kann so die Abtrennung der Restmengen an Phosgen gleichzeitig mit einer Abkühlung des Lösungsmittels verbunden werden. Es wird keine weitere Verwendung von den nach der internen Wärmeintegration verbliebenen Energien beschrieben.

Ebenfalls mit der Herstellung von Isocyanaten befasst sich die europäische Patentanmeldung EP 2 096 102 A1. Es wird ein integriertes Verfahren zur Herstellung von Di- und Polyiscocyanaten der Diphenylmethanreihe (MDI; fortan auch als Gemisch aus Methylendiphenylendiisocyanat - (OCN)C₆H₄-CH₂-C₆H₄(NCO) - und Polymethylenpolyphenylenpolyisocyanat - (OCN)C₆H₄-CH₂-[C₆H₃(NCO)CH₂-]ₓC₆H₄(NCO), wobei x für eine natürliche Zah1 ≥ 1 steht - bezeichnet) beschrieben, bei dem das in der Herstellung der Vorläuferverbindungen der Di- und Polyamine der Diphenylmethanreihe (MDA; fortan auch als Gemisch aus Methylendiphenylendiamin und Polymethylenpolyphenylenpolyamin bezeichnet) anfallende salzhaltige Abwasser zu Chlor elektrolysiert wird, wobei das so gewonnene Chlor zu Phosgen umgesetzt wird, welches dann in der Umsetzung von MDA zu MDI eingesetzt wird. Das Verfahren betrifft also eine stoffliche Integration zwischen den Produktionsanlagen für MDA und MDI. Die Patentanmeldung geht nicht auf *Wärme*integration ein.

Die internationale Patentanmeldung WO 2008/148608 A1 beschreibt die gesamte MDI-Prozesskette ausgehend vom Ausgangsmaterial Benzol, das über die Schritte Nitrierung, Hydrierung, Kondensation mit Formaldehyd und säurekatalysierte Umlagerung und schließlich Phosgenierung in MDI überführt wird. Der Patentanmeldung liegt die technische Aufgabe zugrunde, ein Verfahren zur Herstellung von MDI bereitzustellen, das es ermöglicht, Benzol einer geringeren Reinheit als üblicherweise im Stand der Technik eingesetzt als Ausgangsmaterial zu verwenden. Als Lösung dieser Aufgabe wird vorgeschlagen, Benzol einer geringeren Reinheit als üblicherweise im Stand der Technik eingesetzt als Ausgangsmaterial zu verwenden. Diese geringere Reinheit sei durch einen Gehalt an alkylsubstituierten Aromaten im Benzol von 500 bis 5000 ppm charakterisiert. Wärmeintegration zwischen den einzelnen Verfahrensstufen der MDI-Prozesskette ist nicht Gegenstand dieser Anmeldung.

Großtechnische Produktionsanlagen für chemische Produkte werden heutzutage in Stahlskelett-Bauweise errichtet, um dem Platzmangel, der in schon bestehenden Chemieparks oftmals vorherrscht, entgegenzuwirken. Es ist von Vorteil, verschiedene Stufen einer Verfahrenskette (wie bspw. in der Herstellung verschiedener Isocyanate die Stufen der Nitrierung, der Hydrierung, der optionalen weiteren Umsetzung des Produktes der Hydrierung und der Phosgenierung) an einem Standort in einem Produktionsverbund zu etablieren, um Logistikkosten zu sparen. Die einzelnen Produktionsanlagen eines solchen Produktionsverbunds sind üblicherweise hintereinander angelegt. Die enge Bebauung bringt auch Nachteile mit sich, die wiederum minimiert werden müssen. Nachteilig ist der Platzmangel bei der baulichen Errichtung einzelner Produktionsanlagen in einem Produktionsverbund; es kann zu Problemen bei Instandhaltungsmaßnahmen (z. B. Aufstellung von Kränen) kommen. Sicherheitsaspekte wie die Einhaltung gebotener Mindestabstände von Produktionsanlagen zueinander müssen beachtet werden. Die Bereitstellung von Hilfs- und Einsatzstoffen ist infolge beengter Straßen und Plätze zwischen den Produktionsanlagen unter Umständen problematisch. Die notgedrungen größeren Entfernungen zu zentralen Tanklägern (für Hilfsstoffe, Einsatzstoffe, Produkte) machen die Verwendung längerer Rohrleitungen mit größeren Pumpen erforderlich.

Der Energieverbrauch zur direkten oder indirekten Prozesserwärmung bzw. -kühlung durch Dampf ist im Vergleich zu anderen, insbesondere elektrischen Energieverbrauchern, ein großer Kostenblock beim Betreiben von großtechnischen Produktionsanlagen, sodass Maßnahmen zur Optimierung dieses Energieverbrauches die größten Einsparpotentiale bieten. Die Wärmeintegration auf Basis einer Pinch-Analyse schafft hier Abhilfe. Auf diese Weise kann z. B. die optimale Ausgestaltung der Zulaufvorwärmung/-kühlung zur Reduktion des Energiebedarfs von Kolonnen, der Abhitzedampferzeugung und der Einsatz von Wärmepumpen und Brüdenkompression oder der Erzeugung von Kältemittel in einer Absorptionskältemaschine ermittelt werden. In einer Produktionsanlage vorhandene Restwärme kann an anderen Stellen in demselben Prozess, oder zur Beheizung naheliegender Verwaltungsgebäude eingesetzt werden. Zur Gebäudeheizung kann auch Restwärme genutzt werden, die nicht mehr zur Dampferzeugung geeignet ist. Für Gebäudeheizungen sind relativ niedrige Vorlauftemperaturen ausreichend, so dass selbst Restwärme nutzbar ist, die sich nicht mehr zur Dampferzeugung eignet. Denkbar ist auch, Restwärme zur Erzeugung von Kältemittel in einer Absorptionskältemaschine zu verwenden.

Den heutigen Verfahren des Standes der Technik zur großtechnischen Herstellung von Isocyanaten und deren Vorprodukten gelingt es im Allgemeinen, mit hoher Ausbeute die gewünschten Produkte herzustellen und durch Wärmeintegration *innerhalb* einzelner Produktionsanlagen überschüssige Energien sinnvoll zu nutzen. Häufig geschieht dies derart, dass überschüssige Energien eines Vorgangs innerhalb eines Herstellverfahrens eines chemischen Produkts mittels eines geeigneten Apparats (z. B. eines Wärmeaustauschers) zur Erzeugung von Dampf, insbesondere zur Erzeugung von Wasserdampf, verwendet werden und der so erzeugte Dampf in einem Wärme verbrauchenden Vorgang *desselben Herstellverfahrens* eingesetzt wird (siehe hierzu auch die oben erwähnten Beispiele aus der Patentliteratur). Jedoch verbleiben auch nach maximaler Ausnutzung dieser "internen" Wärmeintegration (d. h. Wärmeintegration innerhalb der Produktionsanlage eines chemischen Produkts) häufig noch überschüssige "Restenergien". Aus solchen Restenergien lässt sich im Allgemeinen lediglich Dampf eines vergleichsweise geringen Drucks und geringer Temperatur (umgangssprachlich auch "Schlappdampf" genannt) erzeugen. Solcher Dampf wird dann entweder lediglich zur Beheizung von Verwaltungsgebäuden genutzt oder die Restenergien gehen ohne jegliche sinnvolle Nutzung in Form von Abwärme verloren oder verursachen sogar noch zusätzliche Kosten bspw. durch erforderliche Luftkühler zur "Energievernichtung".

"Echte" Wärmeintegration mit solchen Restenergien ist im Stand der Technik *zur Herstellung von Isocyanaten* nach vorliegendem Kenntnisstand bisher nicht und im Stand der Technik *allgemein* kaum beschrieben:
Die internationale Patentanmeldung WO 2013/083230 A1 offenbart Wärmeintegration in einem Komplex zur Herstellung von Vinylchlorid (VCM) aus Ethylen, Chlorwasserstoff und Sauerstoff bzw. aus Ethylen und Chlor über das isolierte Zwischenprodukt 1,2-Dichlorethan (DCE). VCM ist als Ausgangsmonomer für die Herstellung von Polyvinylchlorid (PVC) von wirtschaftlicher Bedeutung. Im beschriebenen Verfahren wird die bei der Kondensation des Kopfstromes einer Hochsiederkolonne zur Reinigung des Zwischenproduktes DCE anfallende Wärme zur Gewinnung von Niederdruck-Wasserdampf benutzt, welcher dann beispielsweise für den Betrieb einer Strippkolonne zur Reinigung des VCM oder in verschiedenen Einrichtungen einer nachgeschalteten PVC-Anlage Verwendung findet. Die einzelnen Bestandteile eines VCM-Komplexes oder eines VCM/PVC-Komplexes sind jedoch von ihrer Komplexität her nicht vergleichbar mit den einzelnen Produktionsanlagen eines Isocyanat-Produktionsverbunds. Die chemischen Prozesse sind darüber hinaus völlig verschieden von denen in einem Isocyanat-Produktionsverbund. Wärmeintegration zwischen einzelnen Produktionsanlagen eines Isocyanat-Produktionsverbundes stellt die Betreiber infolge der Vielfalt an unterschiedlichen chemischen Prozessen (exemplarisch seine genannt: Chlorherstellung, Kohlenstoffmonoxidherstellung, Phosgenherstellung, Nitrierung inklusive der Bereitstellung der dazu erforderlichen Salpetersäure, Hydrierung inklusive der Bereitstellung des dazu erforderlichen Wasserstoffs, gegebenenfalls Kondensations- und Umlagerungsreaktionen, Phosgenierung) vor völlig andere Herausforderungen.
Die deutsche Patentanmeldung DE 10 2013 205 492 A1 offenbart, dass bei der Herstellung von Vinylacetat anfallender "Eigendampf" einer Temperatur von üblicherweise 120 °C bis 170 °C und eines Absolutdrucks von üblicherweise 2 bis 8 bar an andere Betriebe eines Werkverbundes abgegeben oder zur Beheizung von Produktrohrleitungen oder Gebäuden eingesetzt werden kann. Alle diese Verwertungen werden als nachteilig beschrieben; um welche Art von Betrieben es sich handelt, die solchen Dampf abnehmen könnten, wird nicht offenbart. Der Anmeldung liegt die Aufgabe zugrunde, diese Art von Verwertung des Eigendampfes zugunsten einer *prozessinternen* Verwertung *verzichtbar zu machen.* Als Lösung wird vorgeschlagen, bestimmt Kolonnen im Prozess der Vinylacetatherstellung mit Füllkörpern auszustatten und es so zu ermöglichen, den Eigendampf für die Energiezufuhr der so ausgestalteten Kolonnen einzusetzen.

Weitere Verbesserungen in der Energieintegration bei Verfahren zur Herstellung von Isocyanaten, durch welche solche Restenergien einer sinnvollen Verwendung *in der Herstellung chemischer Produkte* zugeführt werden können, wären daher wünschenswert. Auch unter Nachhaltigkeits- und Umweltgesichtspunkten sind solche Verbesserungen erstrebenswert.

Dem Vorstehend Gesagten Rechnung tragend ist ein Gegenstand der vorliegenden Erfindung ein **Verfahren zur Herstellung von *m* Isocyanaten in *m* Verfahrensketten,** in denen jeweils ein Isocyanat als Endprodukt über mindestens ein Zwischenprodukt hergestellt wird,
wobei jede der *m* Verfahrensketten in mindestens 2 Produktionsanlagen (100 000-1, 100 000-2, ...) durchgeführt wird, nämlich in einer ersten Produktionsanlage (100 000-1) ausgelegt für die Herstellung des mindestens einen Zwischenprodukts und in einer zweiten Produktionsanlage (100 000-2) ausgelegt für die Herstellung des Isocyanat-Endprodukts, wobei die mindestens 2 Produktionsanlagen (100 000-1, 100 000-2,...,) Bestandteil eines Produktionsverbunds (1 000 000) sind,
wobei in dem Produktionsverbund (1 000 000) insgesamt *n* chemische Produkte, die ausgewählt sind aus der Gruppe bestehend aus Zwischenprodukten, Katalysatoren und Isocyanat-Endprodukten, hergestellt werden, wobei *n* eine natürliche Zahl im Bereich von 2 bis 40, bevorzugt im Bereich von 2 bis 20, besonders bevorzugt im Bereich von 2 bis 15, ist und wobei m eine natürliche Zahl im Bereich von 1 bis *n* ist,
wobei bei der Herstellung jedes der *n* chemischen Produkte mindestens ein Wärme abgebender oder Wärme verbrauchender Vorgang stattfindet und im Verfahren zur Herstellung der m Isocyanate insgesamt mindestens ein Wärme abgebender und mindestens ein Wärme verbrauchender Vorgang stattfinden,
wobei
(i) die in einem Wärme abgebenden Vorgang der Herstellung eines der chemischen Produkte in einer ersten Produktionsanlage (100 000-1) freiwerdende Wärmeenergie mindestens teilweise zur Erzeugung von Dampf, insbesondere von Wasserdampf, eines Drucks von 1,31 bar_{(abs.)} bis 1,91 bar_{(abs.)} und einer Temperatur von 107 °C bis 119 °C genutzt wird;
(ii) der in (i) erzeugte Dampf für die Durchführung eines Wärme verbrauchenden Vorgangs in der Herstellung eines von dem in Schritt (i) hergestellten chemischen Produkt verschiedenen chemischen Produktes eingesetzt wird, wobei die Herstellung dieses von dem in Schritt (i) hergestellten chemischen Produkt verschiedenen chemischen Produktes in einer zweiten Produktionsanlage (100000-2) stattfindet, wobei der in (i) erzeugte Dampf von der Dampf abgebenden Stelle in Schritt (i) über eine Rohrleitung zur Position des Wärme verbrauchenden Vorgangs in Schritt (ii) geleitet wird.

Ein *Produktionsverbund (1000 000)* im Sinne der vorliegenden Erfindung bezeichnet einen Verbund bestehend aus mindestens 2 Produktionsanlagen (100 000-1, 100 000-2, ...), wobei jede Produktionsanlage (100 000-1, 100 000-2, ...) für die Herstellung mindestens eines chemischen Produkts ausgelegt ist. In einem erfindungsgemäßen Produktionsverbund (1 000 000) werden mindestens 2 chemische Produkte hergestellt, nämlich das Isocyanat-Endprodukt und mindestens ein Zwischenprodukt. In dieser einfachsten denkbaren Ausgestaltung der Erfindung gilt *m* = 1 (Herstellung genau eines Isocyanats in genau einer Verfahrenskette), und *n* = 2 (Herstellung von zwei chemischen Produkten, nämlich einem Zwischenprodukt und dem Isocyanat-Endprodukt). Ein Beispiel für eine solche Ausgestaltung der Erfindung ist ein Produktionsverbund (1 000 000) aus einer ersten Produktionsanlage (100 000-1), in der Dinitrotoluol zu Toluylendiamin hydriert wird, und einer zweiten Produktionsanlage (100 000-2), in der Toluylendiamin zu Toluylendiisocyanat phosgeniert wird. Die Zahl *n* bezeichnet die Anzahl der in dem Produktionsverbund (1 000 000) insgesamt *hergestellten* chemischen Produkte. Die Anzahl der tatsächlich im Produktionsverbund (1 000 000) *verarbeiteten* chemischen Produkte ist größer, nämlich mindestens um die Anzahl der *außerhalb des Produktionsverbunds hergestellten* (beispielsweise zugekauften) Ausgangsmaterialien. Es ist auch möglich, bestimmte Hilfsreagenzien von außerhalb bereitzustellen, sodass diese nicht als chemische Produkte *im Sinne der Erfindung* gelten.

Eine Produktionsanlage (100 000) besteht aus den für die Herstellung und erforderlichenfalls Reinigung des in ihr hergestellten chemischen Produkts erforderlichen Einrichtungen. Ein Produktionsverbund (1 000 000) im Sinne der Erfindung zeichnet sich dadurch aus, dass jede ihm zugehörige Produktionsanlage (100000) mit mindestens einer anderen Produktionsanlage (100 000) energetisch gekoppelt ist. Die Anzahl der Produktionsanlagen (100 000) kann kleiner sein als die Anzahl *n* der chemischen Produkte, jedoch nicht größer (ist also maximal gleich *n).*

*Chemische Produkte* im Sinne der Erfindung sind neben den *m* Isocyanat-Endprodukten alle *im Produktionsverbund (1 000 000) hergestellten* Zwischenprodukte der *m* Verfahrensketten und die gegebenenfalls für die einzelnen chemischen Umsetzungen erforderlichen Katalysatoren, insofern diese *im Produktionsverbund (1 000000) hergestellt* werden (z. B. Salzsäure als Umlagerungskatalysator in der Herstellung von Methylendiphenylendiisocyanat, Polymethylenpolyphenylenpolyisocyanat und/oder, Gemischen aus beiden, wenn die Salzsäure nicht komplett zugekauft, sondern zumindest teilweise vor Ort hergestellt wird).

Das erfindungsgemäße Verfahren umfasst *m* Verfahrensketten. Eine solche *Verfahrenskette* im Sinne der Erfindung meint die zur Herstellung des angestrebten Isocyanat-Endprodukts erforderliche Abfolge chemischer Umsetzungen ausgehend von einem bereitgestellten Ausgangsstoff über mindestens ein Zwischenprodukt. Die Wärmeintegration kann dabei *zwischen diesen Verfahrensketten* stattfinden oder *innerhalb einer Verfahrenskette,* dann jedoch *zwischen verschiedenen* Teilschlitten dieser Verfahrenskette (und nicht innerhalb ein und desselben Teilschritts). Beispiele für solche Verfahrensketten im Sinne der Erfindung sind (schematisch und vereinfacht):
(α)
   A) Toluol → Dinitrotoluol;
   B) Dinitrotoluol→ Toluylendiamin;
   C) Toluylendiamin → Toluylendiisocyanat; - *erste Verfahrenskette mit den Teilschritten A) bis C) und dem Isocyanat-Endprodukt Toluylendiisocyanat;*
(β)
   A) Benzol → Nitrobenzol;
   B) Nitrobenzol → Anilin;
   C) Anilin → Gemisch aus Methylendiphenylendiamin und Polymethylenpolyphenylenpolyamin (MDA);
   D) MDA → Gemisch aus Methylendiphenylendiisocyanat und Polymethylenpolyphenylenpolyisocyanat (MDI); - *zweite Verfahrenskette mit den Teilschritten A) bis D) und dem Isocyanat-Endprodukt MDI.*

Eine erfindungsgemäße Wärmeintegration kann dann zwischen der Verfahrenskette (1) und der Verfahrenskette (2) stattfinden oder *zwischen verschiedenen Teilschritten* A) bis C) bzw. A) bis D) innerhalb einer der Verfahrensketten (α) bzw. (β).

Erfindungsgemäß wird mindestens an einer Stelle des Verfahrens bzw. in einer Stelle des Produktionsverbundes *Dampf eines Drucks von 1,31 bar_{(abs.)} bis 1,91 bar_{(abs}.₎ und einer Temperatur von 107 °C bis 119 °C* (im Betriebsjargon als "Schlappdampf" bezeichnet) erzeugt. Geeignete Druckmessgeräte zur Bestimmung des Drucks sind dem Fachmann bekannt. Insbesondere seien hier Membrandrucktransmitter genannt, die sich durch eine hohe Genauigkeit auszeichnen. Solche Geräte werden beispielsweise von der Fa. Wika, Fa. Endress + Hauser oder Fa. Emerson angeboten. Es hat sich gezeigt, dass in Fällen, in denen eine sinnvolle Verwendung von Dampf des oben genannten Temperatur- und Druckbereiches in demjenigen Herstellverfahren eines chemischen Produkts, in welchem der Dampf anfällt, nicht oder nicht vollständig möglich ist, dieser dennoch häufig sinnvoll im Herstellverfahren *eines anderen* chemischen Produkts eingesetzt werden kann. Die erfindungsgemäße Vorgehensweise des *Einsatzes des in (i) erzeugten Dampfes für die Durchführung eines Wärme verbrauchenden Vorgangs in der Herstellung eines von dem in Schritt (i) hergestellten chemischen Produkt verschiedenen chemischen Produktes* umfasst dabei auch den Fall, dass nur ein Teil des erzeugten Dampfes *für die Durchführung eines Wärme verbrauchenden Vorgangs in der Herstellung eines von dem in Schritt (i) hergestellten chemischen Produkt verschiedenen chemischen Produktes* eingesetzt wird und der Rest einer anderen Verwendung zugeführt wird. Erfindungsgemäß bevorzugt ist es, mindestens 30,0 %, besonders bevorzugt mindestens 50,0 %, ganz besonders bevorzugt mindestens 70,0 %, außerordentlich besonders bevorzugt mindestens 90,0 % des in Schritt (i) erzeugten Dampfes zur Durchführung des Wärme verbrauchenden Vorgangs in Schritt (ii) einzusetzen. Ganz außerordentlich bevorzugt ist es, den in Schritt (i) erzeugten Dampf vollständig zur Durchführung des Wärme verbrauchenden Vorgangs in Schritt (ii) einzusetzen.

Die *Rohrleitung* aus Schritt (ii) des erfindungsgemäßen Verfahrens wird in allen Ausführungsformen der Erfindung bevorzugt aus Stahl, bspw. Schwarzstahl oder Edelstahl, gefertigt. Besonders bevorzugt ist Schwarzstahl.

Ein *Wärme abgebender Vorgang* im Sinne der vorliegenden Erfindung kann sowohl eine exotherme chemische Reaktion als auch ein physikalischer Vorgang, wie z. B. das Verflüssigen eines gasförmigen Produkts unter Freisetzung von Kondensationswärme, sein. Dementsprechend kann ein *Wärme verbrauchender Vorgang* im Sinne der vorliegenden Erfindung sowohl eine endotherme chemische Reaktion als auch ein physikalischer Vorgang, wie z. B. die Verdampfung eines flüssigen Ausgangsstoffs oder Zwischenprodukts unter Wärmezufuhr sein. Die Nutzbarmachung des Dampfes in Schritt (ii) des erfindungsgemäßen Verfahrens für die Durchführung des Wärme verbrauchenden Vorgangs kann durch *indirekte Beheizung* (das heißt ohne direkten Kontakt des Dampfes mit den chemischen Verbindungen - Ausgangsstoffe, Zwischenprodukte, Endprodukt, Hilfsreagenzien und dgl. - des wärmeverbrauchenden Vorgangs, z. B. durch Beheizung mittels eines Wärmeaustauschers) oder durch *direkten Dampfeintrag* (das heißt durch Vermischen des Dampfes mit den chemischen Verbindungen - Ausgangsstoffe, Zwischenprodukte, Endprodukt, Hilfsreagenzien und dgl. - des Wärme verbrauchenden Vorgangs, z. B. indem einer Kolonne Strippdampf zugeführt wird), erfolgen.

Als *Dampf* im Sinne der vorliegenden Erfindung eignet sich der Dampf jeglicher Flüssigkeit, die sich mit den vorhandenen Restenergien zu Dampf eines Drucks und einer Temperatur im erfindungsgemäßen Bereich verdampfen lässt, und deren Dampf gegenüber den eingesetzten Werkstoffen von Apparaten und Rohrleitungen hinreichend inert ist. Erfolgt die Nutzbarmachung des Dampfes zur Durchführung des Wärme verbrauchenden Vorgangs durch direkten Dampfeintrag, ist ferner darauf zu achten, dass es nicht zu unerwünschten Reaktionen mit den chemischen Verbindungen des Wärme verbrauchenden Vorgangs kommt, was die Wahl geeigneter Dampfquellen einschränken kann. Erfindungsgemäß besonders bevorzugt ist *Wasserdampf* In allen Ausführungsformen der Erfindung wird daher in dem mindestens einen Wärme abgebenden Vorgang bevorzugt Wasserdampf erzeugt, der zur Durchführung des mindestens einen Wärme verbrauchenden Vorgangs eingesetzt wird, und zwar entweder mittels indirekter Beheizung oder mittels direktem Dampfeintrag. Das dazu erforderliche Wasser kann aus verschiedenen Quellen stammen. Geeignet sind insbesondere Frischwasser aus dem vorhandenen Leitungsnetz, Trinkwasser, vollentsalztes Wasser und Dampfkondensat, wobei Wasserqualitäten mit vernachlässigbarem Salzgehalt wie vollentsalztes Wasser und Dampfkondensat bevorzugt sind, da durch deren Einsatz Kalkablagerungen an Apparaten und Rohrleitungen vermieden werden können.

Es folgt zunächst eine Kurzzusammenfassung verschiedener möglicher **Ausführungsformen der**

### Erfindung:

In einer **ersten Ausführungsform der Erfindung,** die mit allen anderen Ausführungsformen kombiniert werden kann, ist das Isocyanat-Endprodukt ausgewählt aus der Gruppe bestehend aus Methylendiphenylendiisocyanat, Polymethylenpolyphenylenpolyisocyanat, Gemischen aus Methylendiphenylendiisocyanat und Polymethylenpolyphenylenpolyisocyanat, Toluylendiisocyanat, Xylylendiisocyanat, 1,5-Pentandiisocyanat, Hexamethylendiisocyanat, Isophorondiisocyanat und Naphthyldiisocyanat.

In einer **zweiten Ausführungsform der Erfindung,** die mit allen Ausführungsformen, welche die Bildung eines aromatischen Isocyanats umfassen, kombiniert werden kann, wird
in einer ersten Verfahrenskette (α)
A) in einer ersten Verfahrensstufe ein aromatischer Kohlenwasserstoff, insbesondere Toluol, zu einer aromatischen Nitroverbindung, insbesondere Dinitrotoluol, nitriert;
B) die aromatische Nitroverbindung, insbesondere Dinitrotoluol, in einer zweiten Verfahrensstufe durch Hydrierung zu einem aromatischen Amin, insbesondere Toluylendiamin, hydriert;
C) das aromatische Amin, insbesondere Toluylendiamin, in einer dritten Verfahrensstufe durch Phosgenierung zum aromatischen Isocyanat-Endprodukt, insbesondere Toluylendiisocyanat, umgesetzt;
und/oder
in einer zweiten Verfahrenskette (β)
A) in einer ersten Verfahrensstufe ein aromatischer Kohlenwasserstoff, insbesondere Benzol, zu einer aromatischen Nitroverbindung, insbesondere Nitrobenzol, nitriert;
B) die aromatische Nitroverbindung, insbesondere Nitrobenzol, in einer zweiten Verfahrensstufe durch Hydrierung zu einem aromatischen Amin, insbesondere Anilin, hydriert;
C) das aromatische Amin, insbesondere Anilin, in einer dritten Verfahrensstufe zu einem anderen aromatischen Amin, insbesondere Methylendiphenylendiamin, Polymethylenpolyphenylenpolyamin und/oder Gemischen aus Methylendiphenylendiamin und Polymethylenpolyphenylenpolyamin, umgesetzt;
D) das aromatische Amin, insbesondere Methylendiphenylendiamin, Polymethylenpolyphenylenpolyamin und/oder Gemische aus Methylendiphenylendiamin und Polymethylenpolyphenylenpolyamin, in einer vierten Verfahrensstufe durch Phosgenierung zum aromatischen Isocyanat-Endprodukt, insbesondere Methylendiphenylendiisocyanat, Polymethylenpolyphenylenpolyisocyanat und/oder Gemischen aus Methylendiphenyldiisocyanat und Polymethylenpolyphenylenpolyisocyanat, umgesetzt.

In einer **dritten Ausführungsform der Erfindung,** die eine besondere Ausgestaltung der zweiten Ausführungsform ist, umfasst die Phosgenierung gemäß Verfahrensstufe (α)C) und/oder gemäß Verfahrensstufe (β) D) Folgendes:
I) Umsetzung des zu phosgenierenden Amins (2) mit Phosgen (3) in der Flüssigphase und Trennung des erhaltenen Verfahrensproduktes in einen flüssigen, das Roh-Isocyanat und Lösungsmittel enthaltenden Strom (60) und einen gasförmigen, Phosgen und Chlorwasserstoff enthaltenden Strom (70);
II) Trennung des flüssigen Stroms (60) aus Schritt I) in einen flüssigen, Lösungsmittel und Roh-Isocyanat enthaltenden Strom (80) und einen gasförmigen, Phosgen und Chlorwasserstoff enthaltenden Strom (90) in einer Destillationsvorrichtung (2100);
III) Trennung des flüssigen Stroms (80) in einen gasförmigen, Lösungsmittel enthaltenden Strom (110) und einen flüssigen, Roh-Isocyanat enthaltenden Strom (100) in einer Destillationsvorrichtung (2200);
IV) Trennung des gasförmigen Stroms (110) nach dessen mindestens teilweiser Verflüssigung in einem Kondensator (2310), in einen flüssigen, Lösungsmittel enthaltenden Strom (120) und einen gasförmigen, Phosgen enthaltenden Strom (130) in einer Destillationsvorrichtung (2300);
V) Gewinnung eines flüssigen Isocyanat-Stroms (140) aus dem flüssigen Strom (100), wobei ein Nebenkomponenten und ggf. Lösungsmittel enthaltender gasförmiger Strom (150) anfällt, in einer Destillationsvorrichtung (2400), optional umfassend einen Schritt V.1), die Abtrennung von polymeren Isocyanatfraktionen in einer vorgeschalteten Einrichtung zur Polymerabtrennung (2410) als Strom (141).

In einer **vierten Ausführungsform der Erfindung,** die eine besondere Ausgestaltung der dritten Ausführungsform ist, umfasst das Verfahren die Verfahrenskette (β), wobei das in der Verfahrensstufe (β) D) zu phosgenierende Amin ein Gemisch aus Methylendiphenylendiamin und Polymethylenpolyphenylenpolyamin ist, wobei ferner Schritt V.1) durchgeführt wird und so das Isocyanat-Endprodukt in den zwei Strömen 140 und 141 anfällt, wobei Strom 140 Methylendiphenylendiisocyanat und Strom 141 ein Gemisch aus Methylendiphenylendiisocyanat und Polymethylenpolyphenylenpolyisocyanat umfasst.

In einer **fünften Ausführungsform der Erfindung,** die eine besondere Ausgestaltung der vierten Ausführungsform ist, ist der Wärme abgebende Vorgang aus Schritt (i) die mindestens teilweise Verflüssigung des gasförmigen Stroms (110) im Kondensator (2310), und der durch die Wärmeabgabe erzeugte Dampf wird in die Verfahrensstufe (β) C) geführt.

In einer **sechsten Ausführungsform der Erfindung,** die eine besondere Ausgestaltung der fünften Ausführungsform ist, umfasst die Verfahrensstufe (β) C) die folgenden Schritte:
IA) Reaktion von Anilin und Formaldehyd in einem Reaktor (3000) in Abwesenheit eines sauren Katalysators unter Bildung eines Aminals, gefolgt von Trennung des erhaltenen Reaktionsgemisches in eine wässrige und eine organische, das Aminal enthaltende Phase in einer in den Reaktor integrierten Phasentrenneinrichtung oder in einem separaten Phasentrennapparat (4000);
IIA) Reaktion zumindest eines Teils der in Schritt IA) erhaltenen organischen, das Aminal enthaltenden Phase in einem Reaktor (5000) mit Säure, wobei das Aminal zu einem Gemisch aus Methylendiphenylendiamin und Polymethylenpolyphenylenpolyamin reagiert;
oder
IB) Reaktion von Anilin und Säure in einem Reaktor;
IIB) Reaktion zumindest eines Teils des in Schritt IB) erhaltenen Reaktionsgemisches in einem Reaktor mit Formaldehyd zu einem Gemischen aus Methylendiphenylendiamin und Polymethylenpolyphenylenpolyamin;
III) Neutralisation des in Schritt IIA) oder IIB) erhaltenen Reaktionsgemisches in einem Reaktor (6000);
IV) Auftrennen des in Schritt III) erhaltenen neutralisierten Reaktionsgemisches in eine organische Phase, umfassend ein Gemisch aus Methylendiphenylendiamin und Polymethylenpolyphenylenpolyamin, und in eine wässrige Phase in einem Trennbehälter (7000);
V) Waschen der organischen Phase mit Waschflüssigkeit in einem Waschbehälter (8000);
VI) Auftrennen des in Schritt V) erhaltenen Gemisches in eine organische Phase, umfassend ein Gemisch aus Methylendiphenylendiamin und Polymethylenpolyphenylenpolyamin, und in eine wässrige Phase in einem Trennbehälter (7000);
VII) Destillieren der organischen Phase aus Schritt VI) in einer Destillationsapparatur (10000) umfassend eine erste Destillationsstufe (10000-1) zur Abtrennung eines Wasser und Anilin enthaltenden Stromes und eine zweite Destillationsstufe (10000-2) zur weiteren Reinigung des in der ersten Stufe nach Abtrennung des Wasser und Anilin enthaltenden Stromes verbleibenden Verfahrensprodukts unter Erhalt eines Gemisches aus Methylendiphenylendiamin und Polymethylenpolyphenylenpolyamin, wobei in der zweiten Stufe (10000-2) optional Strippdampf zur direkten Wärmeübertragung eingesetzt wird;
VIII) optionale Abtrennung von Methylendiphenylendiamin aus dem in Schritt VII) erhaltenen Gemisch aus Methylendiphenylendiamin und Polymethylenpolyphenylenpolyamin.

In einer **siebten Ausführungsform der Erfindung,** die eine besondere Ausgestaltung der sechsten Ausführungsform ist, umfasst die Verfahrensstufe (β) C) zusätzlich Folgendes:
IX) Aufarbeitung
   - der wässrigen Phase aus Schritt IA) oder
   - der wässrigen Phase aus Schritt IV) oder
   - der wässrigen Phase aus Schritt VI) oder
   - des Wasser und Anilin enthaltenden Stroms aus Schritt VII) oder
   - einer Mischung von zwei oder mehr der vorgenannten wässrigen Verfahrensprodukte
   in einer Abwasseraufarbeitungseinrichtung (11000) umfassend einen Abwassersammelbehälter (11000-1), optional einen Abwassererhitzer (11000-2), einen Trennbehälter (11000-3), optional einen Extraktionsbehälter (11000-4) und eine Abwasserdestillationskolonne (11000-5);
   wobei der wärmeverbrauchende Vorgang aus Schritt (ii) ausgewählt ist aus der Gruppe bestehend aus:
   - der Abtrennung des Wasser und Anilin enthaltenden Stromes aus der organischen Phase aus Schritt VI) in der Destillationsstufe (10000-1),
   - der weiteren Reinigung des in der ersten Destillationsstufe (10000-1) nach Abtrennung des Wasser und Anilin enthaltenden Stromes verbleibenden Verfahrensprodukts in der Destillationsstufe (10000-2), wobei bei Einsatz von Strippdampf zur direkten Wärmeübertragung in (10000-2) dieser Strippdampf den in Schritt (i) erzeugten Dampf umfasst und bevorzugt nur aus dem in Schritt (i) erzeugten Dampf besteht,
   - sofern der Abwassererhitzer (11000-2) vorhanden ist, der Erwärmung dieses Abwassererhitzers (11000-2) und
   - Kombinationen von zwei oder mehr der vorgenannten Vorgänge.

In einer **achten Ausführungsform der Erfindung,** die eine besondere Ausgestaltung der sechsten und siebten Ausführungsform ist, umfasst das Verfahren zusätzlich die Verfahrensstufe der Herstellung von Salzsäure als ein weiteres chemisches Produkt, wobei die Salzsäure durch Absorption von Chlorwasserstoff in Wasser oder 0,1%iger bis 20%iger Salzsäure in einer Absorptionskolonne hergestellt wird, wobei die so hergestellte Salzsäure als Säure in Schritt (β) C) IIA) oder (β) C) IB) eingesetzt wird, wobei der Wärme verbrauchende Vorgang das direkte oder indirekte Beheizen des Sumpfes der Absorptionskolonne aus der Verfahrensstufe der Herstellung von Salzsäure ist.

In einer **neunten Ausführungsform der Erfindung,** die eine besondere Ausgestaltung der zweiten Ausführungsform ist, umfasst die Phosgenierung in Verfahrensstufe (α) C) und/oder in Verfahrensstufe (β) D) Folgendes:
0) Bereitstellung gasförmigen Phosgens (3) durch Verdampfung flüssigen Phosgens;
I) Verdampfung des zu phosgenierenden Amins (2), Umsetzung des verdampften Amins mit gasförmigem Phosgen (3) aus Schritt 0) in der Gasphase, Abbruch der Umsetzung durch Inkontaktbringen mit einem Lösungsmittel-haltigen Strom einer Temperatur unterhalb des Siedepunkts des Amins und Trennung des erhaltenen Verfahrensproduktes in einen flüssigen, das Roh-Isocyanat und Lösungsmittel enthaltenden Strom (60) und einen gasförmigen, Phosgen und Chlorwasserstoff enthaltenden Strom (70);
II) Trennung des flüssigen Stroms (60) aus Schritt I) in einen flüssigen, Lösungsmittel und Roh-Isocyanat enthaltenden Strom (80) und einen gasförmigen, Phosgen und Chlorwasserstoff enthaltenden Strom (90) in einer Destillationsvorrichtung (2100);
III) Trennung des flüssigen Stroms (80) in einen gasförmigen, Lösungsmittel enthaltenden Strom (110) und einen flüssigen, Roh-Isocyanat enthaltenden Strom (100) in einer Destillationsvorrichtung (2200);
IV) Trennung des gasförmigen Stroms (110) nach dessen mindestens teilweiser Verflüssigung in einem Kondensator (2310), in einen flüssigen, Lösungsmittel enthaltenden Strom (120) und einen gasförmigen, Phosgen enthaltenden Strom (130) in einer Destillationsvorrichtung (2300);
V) Gewinnung eines flüssigen Isocyanat-Stroms (140) aus dem flüssigen Strom (100), wobei ein Nebenkomponenten und ggf. Lösungsmittel enthaltender gasförmiger Strom (150) und ein Gemisch aus polymeren Anteilen und monomerem Isocyanat als Strom (143) anfallen, in einer bevorzugt als Trennwandkolonne ausgelegten Destillationsvorrichtung (2400).

In einer **zehnten Ausführungsform der Erfindung,** die eine besondere Ausgestaltung der neunten Ausführungsform ist, umfasst das Verfahren die Verfahrenskette (α), wobei der Wärme verbrauchende Vorgang aus Schritt (ii) mindestens ein Teilschritt der Verdampfung des flüssigen Phosgens in Schritt 0) ist und wobei der Wärme abgebende Vorgang aus Schritt (i) aus der Verfahrensstufe (α)B) stammt.

In einer **elften Ausführungsform** der Erfindung, die eine besondere Ausgestaltung der zweiten Ausführungsform ist, umfasst das Verfahren die Verfahrenskette (α)und die Verfahrenskette (β), wobei
der mindestens eine Wärme abgebende Vorgang aus Schritt (i) Bestandteil der Verfahrenskette (α) und der mindestens eine Wärme verbrauchende Vorgang aus Schritt (ii) Bestandteil der Verfahrenskette (β) ist
oder bei welchem
der mindestens eine Wärme abgebende Vorgang aus Schritt (i) Bestandteil der Verfahrenskette (β) und der mindestens eine Wärme verbrauchende Vorgang aus Schritt (ii) Bestandteil der Verfahrenskette (α) ist.

In einer **zwölften Ausführungsform** der Erfindung, die mit allen anderen Ausführungsformen kombiniert werden kann, wird
- in Schritt (i) der Dampf mittels eines Apparats zur indirekten Wärmeübertragung erzeugt, welcher bevorzugt ausgewählt ist aus der Gruppe bestehend aus Naturumlaufkesseln, Zwangsumlaufkesseln, Zwangsdurchlaufkesseln, Rohrbündelwärmeaustauschern, U-Rohr-Wärmeaustauschern, Doppelrohrschlangenwärmeaustauschern, Plattenwärmeaustauschern, Rohrwärmeaustauschern, Spiralwärmeaustauschern und Einsteckkondensatoren oder
- in Schritt (ii) die für die Durchführung des Wärme verbrauchenden Vorgangs erforderliche Wärmemenge mittels eines Apparats zur indirekten Wärmeübertragung bereitgestellt, welcher bevorzugt ausgewählt ist aus der Gruppe bestehend aus Naturumlaufverdampfern, Zwangsumlaufverdampfern, Einsteckverdampfern, Selbstumlaufverdampfern, Kletterverdampfern, Fallfilmverdampfern, Plattenverdampfern, Wendelrohrverdampfern, Dünnschichtverdampfern, Kurzwegverdampfern, Rohrbündelwärmeaustauschern und Spiralwärmeaustauschern oder
- in Schritt (i) der Dampf mittels eines Apparats zur indirekten Wärmeübertragung erzeugt, welcher bevorzugt ausgewählt ist aus der Gruppe bestehend aus Naturumlaufkesseln, Zwangsumlaufkesseln, Zwangsdurchlaufkesseln, Rohrbündelwärmeaustauschern, U-Rohr-Wärmeaustauschern, Doppelrohrschlangenwärmeaustauschern, Plattenwärmeaustauschern, Rohrwärmeaustauschern, Spiralwärmeaustauschern und Einsteckkondensatoren **und** in Schritt (ii) die für die Durchführung des Wärme verbrauchenden Vorgangs erforderliche Wärmemenge mittels eines Apparats zur indirekten Wärmeübertragung bereitgestellt, welcher bevorzugt ausgewählt ist aus der Gruppe bestehend aus Naturumlaufverdampfern, Zwangsumlaufverdampfern, Einsteckverdampfern, Selbstumlaufverdampfern, Kletterverdampfern, Fallfilmverdampfern, Plattenverdampfern, Wendelrohrverdampfern, Dünnschichtverdampfern, Kurzwegverdampfern, Rohrbündelwärmeaustauschern und Spiralwärmeaustauschern.

In einer **dreizehnten Ausführungsform** der Erfindung, die mit allen anderen Ausführungsformen kombiniert werden kann, wird durch Einstellung
- der Größe des von der Dampf abgebenden Stelle in Schritt (i) zur Position des Wärme verbrauchenden Vorgangs in Schritt (ii) geleiteten Dampfstromes oder
- der Nennweite der in Schritt (ii) eingesetzten Rohrleitung oder
- der Größe des von der Dampf abgebenden Stelle in Schritt (i) zur Position des Wärme verbrauchenden Vorgangs in Schritt (ii) geleiteten Dampfstromes und der Nennweite der Rohrleitung aus Schritt (iii)
eine aus dem Quotienten des Volumenstroms des Dampfes und dem Querschnitt der Rohrleitung berechnete Geschwindigkeit des Dampfstromes in der Rohrleitung im Bereich von 10,0 m/s bis 40,0 m/s bewirkt wird.

In einer **vierzehnten Ausführungsform** der Erfindung, die mit allen anderen Ausführungsformen kombiniert werden kann, weist die Rohrleitung eine Länge von maximal 400 m und einem Innendurchmesser dᵢ im Bereich von 300,0 mm bis 1000 mm auf.

Die zuvor kurz geschilderten Ausführungsformen und weitere mögliche Ausgestaltungen der Erfindung werden im Folgenden näher erläutert. Verschiedene Ausführungsformen können dabei beliebig miteinander kombiniert werden, sofern sich für den Fachmann aus dem Zusammenhang nicht das Gegenteil ergibt.

Als Beispiele für bevorzugt mit dem erfindungsgemäßen Verfahren herstellbare **Isocyanate** seien hier als Di- und Polyisocyanate aromatische Di- und Polyisocyanate, wie z. B. Methylendiphenylendiisocyanat (mMDI) als spezifische Isomere oder als Isomerengemisch, Polymethylenpolyphenylenpolyisocyanat (pMDI), Gemische aus Methylendiphenylendiisocyanat und Polymethylenpolyphenylenpolyisocyanat (MDI), Toluylendiisocyanat (TDI) als reine Isomere oder Isomerengemisch, Isomere des Xylylendiisocyanats (XDI), Isomere des Diisocyanatobenzols, 2,6-Xylolisocyanat, 1,5-Naphthylendiisocyanat (1,5-NDI), Diisocyanate auf Basis von aliphatischen oder cycloaliphatischen Kohlenwasserstoffen mit 2 bis 18 Kohlenstoffatomen, wie z.B. 1,4-Butandiisocyanat, 1,5-Pentandiisocyanat (PDI), 1,6-Hexandiisocyanat (HDI), 1,8-Octandiisocyanat, 1,9-Nonandiisocyanat, 1,10-Decandiisocyanat, 2,2-Dimethylpentan-1,5-diisocyanat, 2-Methyl-1,5-pentandiisocyanat (MPDI), 2,4,4(oder 2,2,4)-Trimethyl-1,6-hexandiisocyanat (TMDI), 1,3- und 1,4-Cyclohexandiisocyanat, 1-Isocyanato-3,3,5-trimethyl-5-isocyanatomethylcyclohexan (IPDI), 2,4-, oder 2,6-Diisocyanato-1-methylcyclohexan (H6-TDI), 1-Isocyanato-1-methyl-4(3)-isocyanatomethylcyclohexan (AMCI), 1,3(und/oder 1,4)-Bis(isocyanatomethyl)cyclohexan, Bis(isocyanatomethyl)norbornan (NBDI), 4,4'(und/oder 2,4')-Diisocyanatodicyclohexyl-methan, und (cyclo)aliphatische Triisocyanate mit bis zu 22 Kohlenstoffatomen, wie z.B. Triisocyanatocyclohexan, Tris(isocyanatomethyl)-cyclohexan, Triisocyanato-methylcyclohexan, 1,8-Diisocyanato-4-(isocyanatomethyl)octan, 1,6,11-Undecantriisocyanat, 1,7-Diisocyanato-4-(3-isocyanatopropyl)heptan, 1,6-Diisocyanato-3-(isocyanatomethyl)-hexan oder 1,3,5-Tris(isocyanatomethyl)-cyclohexan genannt.

Die zu den obigen Polyisocyanaten korrespondierenden Amine sind aromatische Di- und Polyamine, wie z.B. Methylendiphenylendiamin (mMDA) als Isomere oder als Isomerengemisch, Polymethylenpolyphenylenpolyamin (pMDA), Gemische aus Methylendiphenylendiamin und Polymethylenpolyphenylenpolyamin (MDA), Toluylendiamin (TDA) als reine Isomere oder Isomerengemisch, Isomere des Xylylendiamins (XDA), Isomere des Diaminobenzols, 2,6-Xylidin, 1,5-Naphthylendiamin (1,5-NDA), Diamine auf Basis von aliphatischen oder cycloaliphatischen Kohlenwasserstoffen mit 2 bis 18 Kohlenstoffatomen, wie z.B. 1,4-Diaminobutan, 1,5-Diaminopentan (PDA), 1,6-Diaminohexan (HDA), 1,8-Diaminooctan, 1,9-Diaminononan, 1,10-Diaminodecan, 2,2-Dimethyl-1,5-diaminopentan, 2-Methyl-1,5-pentandiamin (MPDA), 2,4,4(oder 2,2,4)-Trimethyl-1,6-diaminohexan (TMDA), 1,3- und 1,4-Diaminocyclohexan, 1-Amino-3,3,5-trimethyl-5-aminomethylcyclohexan (IPDA), 2,4-, oder 2,6-Diamino-1-methylcyclohexan (H6-TDA), 1-Amino-1-methyl-4(3)-aminomethylcyclohexan (AMCA), 1,3(und/oder 1,4)-Bis(aminomethyl)cyclohexan, Bis(aminomethyl)norbornan (NBDA), 4,4'(und/oder 2,4')-Diaminodicyclohexyl-methan, (cyclo)aliphatische Triamine mit bis zu 22 Kohlenstoffatomen, wie z.B. Triaminocyclohexan, Tris(aminomethyl)-cyclohexan, Triamino-methylcyclohexan, 1,8-Diamino-4-(aminomethyl)octan, 1,6,11-Undecantriamin, 1,7-Diamino-4-(3-aminopropyl)heptan, 1,6-Diamino-3-(aminomethyl)-hexan oder 1,3,5-Tris(aminomethyl)-cyclohexan.

Die großtechnische Herstellung der oben angeführten Isocyanate durch Umsetzung der korrespondierenden Amine mit Phosgen ist grundsätzlich seit längerem aus dem Stand der Technik bekannt, wobei die Reaktion in der Gas- oder Flüssigphase sowie diskontinuierlich oder kontinuierlich durchgeführt werden (W. Siefken, Liebigs Ann. 562, 75 - 106 (1949)). Verfahren zur Herstellung von organischen Isocyanaten aus primären Aminen und Phosgen sind bereits mehrfach beschrieben worden, siehe beispielsweise G. Wegener et. al. Applied Catalysis A: General 221 (2001), Seiten 303 bis 335, Elsevier Science B.V. sowie Ullmanns Enzyklopädie der technischen Chemie, 4. Aufl. (1977), Band 13, Seiten 351 bis 353.

MDI und TDI sind bezüglich der Herstellungsvolumina weltweit die größten Isocyanat-Produkte.

Die moderne großtechnische Herstellung von MDI erfolgt kontinuierlich, und die Reaktionsführung erfolgt bevorzugt als adiabate Phosgenierung wie in EP 1 616 857 B2 und EP 1 873 142 B1 beschrieben. Die Aufarbeitung des Roh-MDI ist beispielhaft in US 5 136 087 (B), EP 1 854 783 A2, EP 1 475 367 B1 oder auch in EP 1 686 112 A1 beschrieben. MDI fällt zunächst als Gemisch aus Methylendiphenylendiisocyanat und Polymethylenpolyphenylenpolyisocyanat an. Unabhängig davon, ob aus diesem Gemisch reine Monomer-Anteile (mMDI) abgetrennt werden oder nicht, gilt MDI als *ein* (1) chemisches Produkt im Sinne der Erfindung.

Die kontinuierliche Herstellung von TDI erfolgt großtechnisch in der Gasphase, wie z. B. in EP-A-2 196 455, EP-A-0 289 840, EP-A-0 570 799, EP-B-1 935 875 und EP-B-1 935 876 beschrieben, oder in der Flüssigphase, wie z. B. in EP 0 322 647 B1, WO 2013/139703 A1, EP 314 985 B1, EP 1 371 636 B1, EP 1 371 635 B1 und EP 1 413 571 B1 beschrieben.

Die Vorstufe von MDI ist MDA. MDA wiederum wird durch Kondensation von Anilin und Formaldehyd hergestellt. Anilin wird durch Hydrierung von Nitrobenzol erhalten. Nitrobenzol wiederum entsteht durch Nitrierung von Benzol mit Salpetersäure, wobei Benzol die petrochemische Basis zur Herstellung von MDI über die einzelnen Zwischenstufen darstellt. Alternativ kann Anilin auch durch biochemische Prozesse gewonnen werden wie in WO 2015/124686 A1 und WO 2015/124687 A1 sowie den noch unveröffentlichten Patentanmeldungen EP 15195527.5 und EP 16157777.0 beschrieben.

Die kontinuierliche oder diskontinuierliche Herstellung von MDA ist z. B. in EP 1 616 890 A1, US-A-5286760, EP-A-451442 und WO-A-99/40059 offenbart.

Die kontinuierliche Herstellung von Anilin in isothermer oder adiabater Fahrweise erfolgt im großtechnischen Maßstab in der Regel durch katalytische Hydrierung von Nitrobenzol wie z. B. in GB 1 452 466 A1, EP 0 011 090 A1 oder EP 0 944 578 A2 (isotherme Fahrweise) und in EP 0 696 574 B1, EP 0 696 573 B1, EP 1 882 681 A1 (adiabate Fahrweise) beschrieben. Neben den genannten Verfahren mit stationären Katalysatorbetten sind auch solche mit fluidisierten Katalysatorbetten z. B. in DE 1114820 B, DE 1133394 B oder WO 2008/034770 A1 beschrieben.

Die heute gängigen Verfahren zur großtechnischen Herstellung von Nitrobenzol entsprechen im Wesentlichen dem Konzept der adiabaten Nitrierung von Benzol durch ein Gemisch von Schwefel- und Salpetersäure, welches im Allgemeinen als Mischsäure bezeichnet wird. Ein solches Verfahren wurde erstmals in US 2,256,999 beansprucht und ist in heutigen Ausführungsformen beispielsweise in EP 0 436 443 B1, EP 0 771 783 B1 und US 6 562 247 B2 beschrieben. Auch sind isotherme Verfahren zur Nitrierung von Benzol mit Mischsäure bekannt, wie beispielsweise in EP 0 156 199 B1 beschrieben.

Die Vorstufe von TDI ist TDA. TDA wiederum wird durch Hydrierung von Dinitrotoluol (DNT) erhalten. DNT wiederum entsteht durch Nitrierung von Toluol, was die petrochemische Basis zur Herstellung von TDI über die einzelnen Zwischenstufen darstellt.

Die moderne, kontinuierliche Herstellung von TDA in isothermer und/oder adiabater Fahrweise erfolgt im großtechnischen Maßstab in der Regel durch katalytische Hydrierung von DNT wie z. B. ausführlich in WO 2011/086050 A1 und darin zitierten Literaturstellen beschrieben.

Die Herstellung des Dinitrotoluols durch Nitrierung von Toluol mit Nitriersäure (Gemisch von Salpetersäure und Schwefelsäure) war bereits Gegenstand zahlreicher Veröffentlichungen und Patentanmeldungen (Ullmanns Enzyklopedie der technischen Chemie, 4.Auflage, Band 17, Seite 391 ff, 1979, Verlag Chemie Weinheim / New York). Die industrielle Herstellung erfolgt vorwiegend isotherm mit Salpetersäure in Gegenwart von Schwefelsäure als Katalysator in kontinuierlicher Fahrweise, wie beispielsweise in H. Hermann, J. Gebauer, P. Konieczny, "Industrial Nitration of Toluene to Dinitrotoluene" in ACS-Symposium, Series 623, 234-249, 1996, ed. L.F.Albright, R.V.C Carr, R.J. Schmitt beschrieben.

Die Herstellung von Salpetersäure ist in zahlreichen Veröffentlichungen und Patentanmeldungen beschrieben. Die Anlage ist üblicherweise so dimensioniert, dass verschiedene Verfahrensketten wie z. B. eine TDI-Verfahrenskette und eine MDI-Verfahrenskette aus einer Salpetersäure-Anlage beliefert werden können.

Erfindungsgemäß besonders bevorzugt ist das Isocyanat ausgewählt aus der Gruppe bestehend aus Methylendiphenylendiisocyanat, Polymethylenpolyphenylenpolyisocyanat, Gemischen aus Methyl endiphenylendiisocyanat und Polymethylenpolyphenylenpolyisocyanat, Toluylendiisocyanat, Xylylendiisocyanat, 1,5-Pentandiisocyanat, Hexamethylendiisocyanat, Isophorondiisocyanat und Naphthyldiisocyanat.

Für **aromatische Isocyanate** gilt vorzugsweise, dass
in einer ersten Verfahrenskette 1(α)
A) in einer ersten Verfahrensstufe ein aromatischer Kohlenwasserstoff, insbesondere Toluol, zu einer aromatischen Nitroverbindung, insbesondere Dinitrotoluol, nitriert wird;
B) die aromatische Nitroverbindung, insbesondere Dinitrotoluol, in einer zweiten Verfahrensstufe durch Hydrierung zu einem aromatischen Amin, insbesondere Toluylendiamin, hydriert wird;
C) das aromatische Amin, insbesondere Toluylendiamin, in einer dritten Verfahrensstufe durch Phosgenierung zum aromatischen Isocyanat-Endprodukt, insbesondere Toluylendiisocyanat, umgesetzt wird;
und/oder
in einer zweiten Verfahrenskette (β)
A) in einer ersten Verfahrensstufe ein aromatischer Kohlenwasserstoff, insbesondere Benzol, zu einer aromatischen Nitroverbindung, insbesondere Nitrobenzol, nitriert wird;
B) die aromatische Nitroverbindung, insbesondere Nitrobenzol, in einer zweiten Verfahrensstufe durch Hydrierung zu einem aromatischen Amin, insbesondere Anilin, hydriert wird;
C) das aromatische Amin, insbesondere Anilin, in einer dritten Verfahrensstufe zu einem anderen aromatischen Amin, insbesondere Methylendiphenylendiamin, Polymethylenpolyphenylenpolyamin und/oder Gemischen aus Methylendiphenylendiamin und Polymethylenpolyphenylenpolyamin, umgesetzt wird;
D) das aromatische Amin, insbesondere Methylendiphenylendiamin, Polymethylenpolyphenylenpolyamin und/oder Gemische aus Methylendiphenylendiamin und Polymethylenpolyphenylenpolyamin, in einer vierten Verfahrensstufe durch Phosgenierung zum aromatischen Isocyanat-Endprodukt, insbesondere Methylendiphenylendiisocyanat, Polymethylenpolyphenylenpolyisocyanat und/oder Gemischen aus Methylendiphenylendiisocyanat und Polymethylenpolyphenylenpolyisocyanat, umgesetzt wird.

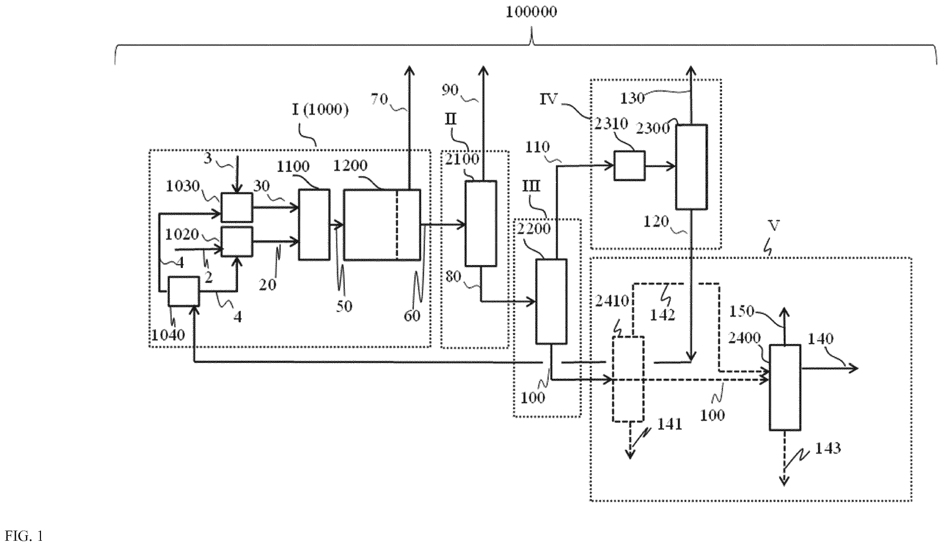

In einer bevorzugten Ausgestaltung der Erfindung betreffend die Produktion aromatischer Isocyanate findet die Phosgenierung **in der Flüssigphase** statt (vgl. hierzu auch FIG. 1). Insbesondere betrifft diese Ausführungsform ein Verfahren, bei welchem die Phosgenierung gemäß Verfahrensstufe (α) C) und/oder gemäß Verfahrensstufe (β) D) Folgendes umfasst:
I) Umsetzung des zu phosgenierenden Amins (2) mit Phosgen (3) in der Flüssigphase und Trennung des erhaltenen Verfahrensproduktes in einen flüssigen, das Roh-Isocyanat und Lösungsmittel enthaltenden Strom (60) und einen gasförmigen, Phosgen und Chlorwasserstoff enthaltenden Strom (70);
II) Trennung des flüssigen Stroms (60) aus Schritt I) in einen flüssigen, Lösungsmittel und Roh-Isocyanat enthaltenden Strom (80) und einen gasförmigen, Phosgen und Chlorwasserstoff enthaltenden Strom (90) in einer Destillationsvorrichtung (2100);
III) Trennung des flüssigen Stroms (80) in einen gasförmigen, Lösungsmittel enthaltenden Strom (110) und einen flüssigen, Roh-Isocyanat enthaltenden Strom (100) in einer Destillationsvorrichtung (2200);
IV) Trennung des gasförmigen Stroms (110) nach dessen mindestens teilweiser Verflüssigung in einem Kondensator (2310), in einen flüssigen, Lösungsmittel enthaltenden Strom (120) und einen gasförmigen, Phosgen enthaltenden Strom (130) in einer Destillationsvorrichtung (2300);
V) Gewinnung eines flüssigen Isocyanat-Stroms (140) aus dem flüssigen Strom (100), wobei ein Nebenkomponenten und ggf. Lösungsmittel enthaltender gasförmiger Strom (150) anfällt, in einer Destillationsvorrichtung (2400), optional umfassend einen Schritt V.1), die Abtrennung von polymeren Isocyanatfraktionen in einer vorgeschalteten Einrichtung zur Polymerabtrennung (2410) als Strom (141).

Eine bevorzugte apparative Ausgestaltung der Verfahrensstufe der Phosgenierung dieser Ausführungsform zeigt FIG. 1. Vorrichtungen zur Aufarbeitung der gasförmigen Ströme (70, 90, 130, 150) sind der Einfachheit halber nicht gezeigt, weil sie für das Verständnis des erfindungsgemäßen Verfahrens nicht von Belang sind. Ein- bis dreistellige Bezugszeichen bezeichnen Stoffströme mit der oben genannten Bedeutung. Alle Vorrichtungen sind mit vierstelligen Bezugszeichen versehen. Es bedeuten:
1000: Reaktionsstrecke;
1020: Einrichtung zur Bereitstellung eines Amins (2) in Form einer Aminlösung (20) in einem Lösungsmittel (4);
1030: Einrichtung zur Bereitstellung von Phosgen (3) in Form einer Phosgenlösung (30) in einem Lösungsmittel (4);
1040: Einrichtung zur Bereitstellung eines Lösungsmittels (4);
1100: Mischeinrichtung zur Vermischung von Aminlösung (20) mit Phosgenlösung (20) und optional weiterem Lösungsmittel (4);
1200: Reaktionsraum zur Durchführung der Phosgenierung, dem optional eine Abscheideeinrichtung (1210) nachgeschaltet ist, wobei der Reaktionsraum oder die Abscheidevorrichtung mit Abfuhrleitungen für den flüssigen Strom (60) und den gasförmigen Strom (70) versehen sind;
2100: Destillationsvorrichtung ("Entpllosgenierkolonne") zur Auftrennung des flüssigen Stroms (60) in einen flüssigen Strom (80) und einen gasförmigen Strom (90);
2200: Destillationsvorrichtung ("Lösungsmittelkolonne") zur Trennung des flüssigen Stroms (80) in einen gasförmigen Strom (110) und einen flüssigen Strom (100);
2300: Destillationsvorrichtung ("Lösungsmittelstripper") zur Trennung des gasförmigen Stroms (110) nach dessen Verflüssigung in einem Kondensator (2310) in einen flüssigen Strom (120) und einen gasförmigen Strom (130);
2400: Destillationsvorrichtung (2400) zur Gewinnung eines flüssigen Isocyanat-Stroms (140) aus dem flüssigen Strom (100), wobei ein Nebenkomponenten und ggf. Lösungsmittel enthaltender gasförmiger Strom (150) anfällt,
   optional umfassend
2410: eine vorgeschaltete Einrichtung zur Polymerabtrennung ("Polymerabtrennung", PMA) zur Abtrennung von polymeren Isocyanatfraktionen (141).

Diese Ausführungsform des erfindungsgemäßen Verfahrens eignet sich insbesondere für die Herstellung von Methylendiphenylendiisocyanat und eines Gemisches aus Methylendiphenylendiisocyanat und Polymethylenpolyphenylenpolyisocyanat in der Verfahrenskette (β). In dieser Ausgestaltung des Verfahrens ist dann das zur Phosgenierung vorgesehene Amin ein Gemisch aus Methylendiphenylendiamin und Polymethylenpolyphenylenpolyamin, wobei Schritt V.1) (sog. "Polymerabtrennung") durchgeführt wird und so das Isocanat-Endprodukt in den zwei Strömen 140 und 141 anfällt, wobei Strom 140 Methylendiphenylendiisocyanat und Strom 141 ein Gemisch aus Methylendiphenylendiisocyanat und Polymethylenpolyphenylenpolyisocyanat ist.

In dieser Ausführungsform des erfindungsgemäßen Verfahrens ist der Wärme abgebende Vorgang aus Schritt (i) bevorzugt die mindestens teilweise Verflüssigung des gasförmigen Stroms (110) im Kondensator (2310), wobei der durch die Wärmeabgabe erzeugte Dampf in die Verfahrensstufe der Herstellung des zu phosgenierenden Amins, also die MDA-Herstellung (Verfahrensstufe (β) C)), geführt wird.

Die MDA-Verfahrensstufe (β) C) umfasst dabei bevorzugt die folgenden Schritte:
IA) Reaktion von Anilin (5) und Formaldehyd (6) in einem Reaktor (3000) in Abwesenheit eines sauren Katalysators unter Bildung eines Aminals, gefolgt von Trennung des erhaltenen Reaktionsgemisches in eine wässrige (8) und eine organische, das Aminal enthaltende Phase (9) in einer in den Reaktor integrierten Phasentrenneinrichtung oder in einem separaten Phasentrennapparat (4000);
IIA) Reaktion zumindest eines Teils der in Schritt IA) erhaltenen organischen, das Aminal enthaltenden Phase in einem Reaktor (5000) mit Säure (10), wobei das Aminal zu einem Gemisch (11) aus Methylendiphenylendiamin und Polymethylenpolyphenylenpolyamin reagiert;
- sog. *Aminalverfahren -*
oder
IB) Reaktion von Anilin (5) und Säure (10) in einem Reaktor;
IIB) Reaktion zumindest eines Teils des in Schritt IB) erhaltenen Reaktionsgemisches in einem Reaktor mit Formaldehyd (6) zu einem Gemisch (11) aus Methylendiphenylendiamin und Polymethylenpolyphenylenpolyamin;
- sog. *Neutralisationsverfahren* -
III) Neutralisation des in Schritt IIA) oder IIB) erhaltenen Reaktionsgemisches (11) in einem Reaktor (6000);
IV) Auftrennen des in Schritt III) erhaltenen neutralisierten Reaktionsgemisches (12) in eine organische Phase (13), umfassend ein Gemisch aus Methylendiphenylendiamin und Polymethylenpolyphenylenpolyamin, und in eine wässrige Phase (14) in einem Trennbehälter (7000);
V) Waschen der organischen Phase (13) mit Waschflüssigkeit (15) in einem Waschbehälter (8000);
VI) Auftrennen des in Schritt V) erhaltenen Gemisches (16) in eine organische Phase (17), umfassend ein Gemisch aus Methylendiphenylendiamin und Polymethylenpolyphenylenpolyamin, und in eine wässrige Phase (18) in einem Trennbehälter (7000);
VII) Destillieren der organischen Phase aus Schritt VI) in einer Destillationsapparatur (10000) umfassend eine erste Destillationsstufe (10000-1) zur Abtrennung eines Wasser und Anilin enthaltenden Stromes (19) und eine zweite Destillationsstufe (10000-2) zur weiteren Reinigung des in der ersten Stufe nach Abtrennung des Wasser und Anilin enthaltenden Stromes verbleibenden Verfahrensprodukts (20) unter Abtrennung eines (feuchten) Anilin enthaltenden Stromes (22) und unter Erhalt eines Gemisches (21) aus Methylendiphenylendiamin und Polymethylenpolyphenylenpolyamin, wobei in der zweiten Stufe (10000-2) optional Strippdampf zur direkten Wärmeübertragung eingesetzt wird;
VIII) optionale Abtrennung von Methylendiphenylendiamin aus dem in Schritt VII) erhaltenen Gemisch (21) aus Methylendiphenylendiamin und Polymethylenpolyphenylenpolyamin.

Besonders bevorzugt schließt sich an Schritt VIII) Folgendes an:
IX) Aufarbeitung
- der wässrigen Phase aus Schritt IA) oder
- der wässrigen Phase aus Schritt IV) oder
- der wässrigen Phase aus Schritt VI) oder
- des Wasser und Anilin enthaltenden Stroms aus Schritt VII) oder
- einer Mischung von zwei oder mehr der vorgenannten wässrigen Verfahrensprodukte in einer Abwasseraufarbeitungseinrichtung (11000) umfassend einen Abwassersammelbehälter (11000-1), optional einen Abwassererhitzer (11000-2), einen Trennbehälter (11000-3), optional einen Extraktionsbehälter (11000-4), und eine Abwasserdestillationskolonne (11000-5; z. B. ein Abwasserstripper);
wobei der Wärme verbrauchende Vorgang aus Schritt (ii) ausgewählt ist aus der Gruppe bestehend aus:
- der Abtrennung des Wasser und Anilin enthaltenden Stromes aus der organischen Phase aus Schritt VI) in der Destillationsstufe (10000-1),
- der weiteren Reinigung des in der ersten Destillationsstufe (10000-1) nach Abtrennung des Wasser und Anilin enthaltenden Stromes verbleibenden Verfahrensprodukts in der Destillationsstufe (10000-2), wobei bei Einsatz von Strippdampf zur direkten Wärmeübertragung in (10000-2) dieser Strippdampf den in Schritt (i) erzeugten Dampf umfasst und bevorzugt nur aus dem in Schritt (i) erzeugten Dampf besteht,
- sofern der Abwassererhitzer (11000-2) vorhanden ist, der Erwärmung des Abwassererhitzers (11000-2) und
- Kombinationen von zwei oder mehr der vorgenannten Vorgänge.

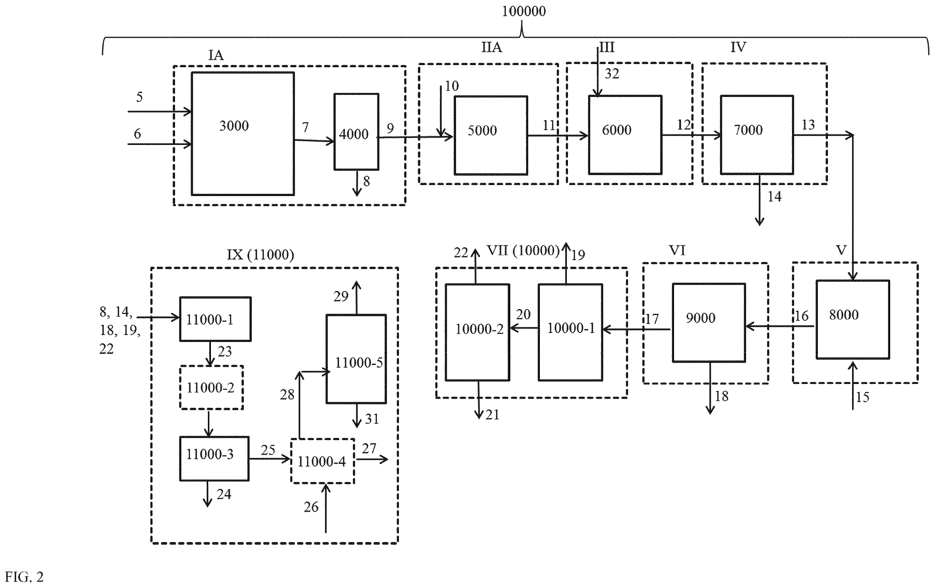

Eine bevorzugte apparative Ausgestaltung der Verfahrensstufe der Herstellung des zu phosgenierenden Amins dieser Ausführungsform unter Verwendung des Aminalverfahrens zeigt FIG. 2 (ohne Apparate für den optionalen Schritt VIII)). Ein- bis zweistellige Bezugszeichen bezeichnen Stoffströme mit der oben genannten Bedeutung. Alle Vorrichtungen sind mit vier- oder fünfstelligen Bezugszeichen versehen. Es bedeuten:
3000: Reaktor;
4000: Trennbehälter;
5000: Reaktor zur Reaktion der aus 4000 erhaltenen organischen Phase mit Säure;
6000: Reaktor zur Neutralisation des Reaktionsgemisches aus 5000;
7000: Trennbehälter zum Auftrennen des neutralisierten Reaktionsgemisches aus 5000 in eine organische Phase und eine wässrige Phase;
8000: Waschbehälter zum Waschen der organischen Phase aus 7000 mit Waschflüssigkeit;
9000: Trennbehälter zum Auftrennen des Gemisches aus 8000 in eine organische und eine wässrige Phase;
10000: Destillationseinrichtung zum Destillieren der organischen Phase aus 9000;
   10000-1: erste Destillationsstufe ("Vorverdampfer");
   10000-2: zweite Destillationsstufe ("MDA-Kolonne");
11000: Abwasseraufarbeitungseinrichtung zur Aufarbeitung des wässrigen Verfahrensprodukts aus 4000 und/oder aus 7000 und/oder aus 9000 und/oder aus 10000-1;
   11000-1: Abwassersammelbehälter zur Aufnahme der genannten Abwasserströme;
   11000-2: optionaler Abwassererhitzer zum Erhitzen der vereinigten Abwasserströme (23);
   11000-3: Trennbehälter zur Abtrennung organischer, Anilin enthaltender Bestandteile (24) aus (23);
   11000-4: optionaler Extraktionsbehälter, der ein- oder mehrstufig ausgelegt sein kann, zur Extraktion des nach der Abtrennung organischer, Anilin enthaltender Bestandteile (24) aus (23) verbleibenden wässrigen Verfahrensprodukts (25) mit Anilin (26) unter Abtrennung einer organischen Phase (27) enthaltend Anilin und MDA;
   11000-5: Abwasserdestillationskolonne zur Reinigung des wässrigen Verfahrensprodukts aus 11000-3 oder aus 11000-4, (25) bzw. (28) unter Erhalt gereinigten Abwassers (29) und eines Anilin enthaltenden Strom (31).

In dieser Ausführungsform der Erfindung wird also überschüssige Energie aus dem MDI-Herstellverfahren (Verfahrensstufe der Phosgenierung), in Form von Dampf, konkret in Form des Dampfes aus dem Dampferzeuger des Kondensators (2310) der Destillationsvorrichtung (2200) (der *"Lösungsmittelkolonne*"), über eine Rohrleitung dem MDA-Prozess (der Herstellung des zu phosgenierenden Amins) zugeführt, und dort beispielsweise als Strippdampf in der Destillationsstufe (8000-2) oder als Heizdampf für die Destillationsstufe (10000-1) (sog. *"Vorverdampfer der MDA-Kolonne*") oder als Heizdampf für die Erwärmung des Abwassererhitzers (10000-2) oder als Heizdampf für die Abwasserdestillationskolonne (11000-5; "Anilinstripper") genutzt. Es ist jedoch nicht zwingend erforderlich, den im Dampferzeuger des Kondensators (2310) gewonnenen Dampf *komplett* der MDA-Stufe zuzuführen; ein Teil kann auch anderweitig eingesetzt werden.

In einer weiteren Ausführungsform der Erfindung wird überschüssige Energie aus dem MDI-Herstellverfahren (Verfahrensstufe der Phosgenierung) in einem Verfahren zur Herstellung von Salzsäure als ein weiteres chemisches Produkt durch Absorption von Chlorwasserstoff in Wasser oder verdünnter (0,1%iger bis 20%iger, massenbezogen) Salzsäure eingesetzt. Dabei wird die so hergestellte Salzsäure als Säure in Schritt (β) C) IIA) oder (β) C) IB) eingesetzt,
wobei der Wärme abgebende Vorgang aus Schritt (i) die mindestens teilweise Verflüssigung des gasförmigen Stroms (110) im Kondensator (2310) ist und wobei der Wärme verbrauchende Vorgang das direkte oder indirekte Beheizen des Sumpfes der Absorptionskolonne aus der Verfahrensstufe der Herstellung der Salzsäure ist.

In einer weiteren bevorzugten Ausgestaltung der Erfindung betreffend die Produktion aromatischer Isocyanate, insbesondere die Produktion von TDI, findet die Phosgenierung **in der Gasphase** statt. Insbesondere betrifft diese Ausführungsform ein Verfahren, bei welchem die Phosgenierung in Verfahrensstufe (α) C) und/oder in Verfahrensstufe (β) D) Folgendes umfasst:
0) Bereitstellung gasförmigen Phosgens (3) durch Verdampfung flüssigen Phosgens;
I) Verdampfung des zu phosgenierenden Amins (2), Umsetzung des verdampften Amins mit gasförmigem Phosgen (3) aus Schritt 0) in der Gasphase, Abbruch der Umsetzung durch Inkontaktbringen mit einem Lösungsmittel-haltigen Strom einer Temperatur unterhalb des Siedepunkts des Amins (sog. "Quenche") und Trennung des erhaltenen Verfahrensproduktes in einen flüssigen, das Roh-Isocyanat und Lösungsmittel enthaltenden Strom (60) und einen gasförmigen, Phosgen und Chlorwasserstoff enthaltenden Strom (70);
II) Trennung des flüssigen Stroms (60) aus Schritt I) in einen flüssigen, Lösungsmittel und Roh-Isocyanat enthaltenden Strom (80) und einen gasförmigen, Phosgen und Chlorwasserstoff enthaltenden Strom (90) in einer Destillationsvorrichtung (2100);
III) Trennung des flüssigen Stroms (80) in einen gasförmigen, Lösungsmittel enthaltenden Strom (110) und einen flüssigen, Roh-Isocyanat enthaltenden Strom (100) in einer Destillationsvorrichtung (2200);
IV) Trennung des gasförmigen Stroms (110) nach dessen mindestens teilweiser Verflüssigung in einem Kondensator (2310), in einen flüssigen, Lösungsmittel enthaltenden Strom (120) und einen gasförmigen, Phosgen enthaltenden Strom (130) in einer Destillationsvorrichtung (2300);
V) Gewinnung eines flüssigen Isocyanat-Stroms (140) aus dem flüssigen Strom (100), wobei ein Nebenkomponenten und ggf. Lösungsmittel enthaltender gasförmiger Strom (150) und ein Gemisch aus polymeren Anteilen ("Rückstand") und monomerem Isocyanat (TDI) als Strom (143) anfallen, in einer bevorzugt als Trennwandkolonne ausgelegten Destillationsvorrichtung (2400).

Insbesondere TDI ist mit dieser Ausführungsform der Erfindung vorteilhaft in der Verfahrenskette (α) herstellbar. In dieser Ausführungsform ist der Wärme verbrauchende Vorgang aus Schritt (ii) bevorzugt mindestens ein Teilschritt der Verdampfung des flüssigen Phosgens in Schritt 0). Bevorzugt erfolgt diese Verdampfung, indem flüssiges Phosgen in einer ersten Stufe vorgewärmt und in einer zweiten Stufe weiter aufgeheizt und verdampft wird. Insbesondere die erste Stufe dieser zweistufigen Verdampfung wird als der mindestens eine Wärme verbrauchende Vorgang im Sinne der vorliegenden Erfindung ausgestaltet, d. h. die Vorerwärmung des flüssigen Phosgens erfolgt mittels indirekter Beheizung durch den in Schritt (i) erzeugten Dampf. Der Schritt 0) kann in bevorzugten Ausgestaltungen der Erfindung neben der reinen Verdampfung des Phosgens noch weitere Schritte umfassen. So ist es möglich, den zunächst erhaltenen gasförmigen Phosgenstrom in eine Kolonne (sog. Phosgendesorptionskolonne) zu leiten, in welcher Verunreinigungen wie CO oder inerte Gase entfernt werden, und den gereinigten Phosgengasstrom in Schritt I) einzusetzen.

Der in der Verdampfung des flüssigen Phosgens in Schritt 0) eingesetzte Dampf stammt bevorzugt aus der Verfahrensstufe der Herstellung des zu phosgenierenden Amins in Verfahrensstufe (α) B), also insbesondere der TDA-Herstellung. Besonders bevorzugt ist der Wärme abgebende Vorgang aus Schritt (i) in dieser Ausführungsform die (exotherme) Hydrierung der aromatischen Nitroverbindung (insbesondere Dinitrotoluol) zu dem aromatischen Amin (insbesondere TDA), wobei die freiwerdende Reaktionswärme zur Erzeugung von Dampf eines Drucks von 1,31 bar_{(abs.)} bis 1,91 bar_{(abs.)} und einer Temperatur von 107 °C bis 119 °C genutzt wird. Es ist jedoch auch möglich, diesen Dampf aus einer Anlage einer anderen Verfahrenskette zu entnehmen, beispielsweise dem Verfahrensschritt der Flüssigphasen-Phosgenierung von MDA zu MDI der MDI-Prozesskette.

Auch für die Herstellung des Isocyanats in der Gasphase kann auf FIG. 1 verwiesen werden, wobei dann allerdings auf die Einrichtung (2410) verzichtet wird (d. h. der Strom 100 wird direkt in die Destillationsvorrichtung 2400 geleitet; vgl. die gestrichelt gezeichnete Leitung für Strom 100 in FIG. 1), und die Destillationsvorrichtung (2400) wird bevorzugt als Trennwandkolonne ausgestaltet. Gereinigtes Isocyanat (140), also insbesondere Rein-TDI, wird der Kolonne (2400) als Seitenstrom entnommen, während im Sumpf der Kolonne ein Gemisch aus polymeren Anteilen ("Rückstand") und monomerem Isocyanat (insbesondere TDI) als Strom (143) anfällt. In dieser Ausführungsform ist also die Abtrennung polymerer Isocyanatfraktionen apparativ in die Destillationsvorrichtung (2400) integriert. Schritt 0) (Verdampfung des Phosgens) und die Verdampfung des Amins sind aus Gründen der zeichnerischen Vereinfachung nicht eingezeichnet. Auch ist es in dieser Ausführungsform nicht zwingend, Phosgen und Amin *in Form von Lösungen* einzusetzen. Es ist möglich, Lösungsmittel (4) dem in Strömungsrichtung hinteren Teil des Reaktionsraums (1200) erstmalig zum Reaktionsabbruch ("Quenche") zuzuführen (ebenfalls nicht gezeigt in FIG. 1).

Bisher wurden konkrete Beispiele für Wärmeintegration *innerhalb* einer der Verfahrensketten (α) oder (β) aufgezeigt. Es ist jedoch ebenfalls möglich, beide Verfahrensketten in demselben Produktionsverbund durchzuführen und die Wärmeintegration zwischen denselben vorzunehmen. In dieser Ausführungsform umfasst das erfindungsgemäße Verfahren demnach Verfahrenskette (α) *und* Verfahrenskette (β), wobei
der mindestens eine Wärme abgebende Vorgang aus Schritt (i) Bestandteil der Verfahrenskette (α) und der mindestens eine Wärme verbrauchende Vorgang aus Schritt (ii) Bestandteil der Verfahrenskette (β) ist
oder wobei
der mindestens eine Wärme abgebende Vorgang aus Schritt (i) Bestandteil der Verfahrenskette (β) und der mindestens eine Wärme verbrauchende Vorgang aus Schritt (ii) Bestandteil der Verfahrenskette (α) ist.

Eine konkrete Ausgestaltung dieser Verfahrensweise ist in Beispiel 3 gezeigt.

In allen Ausführungsformen des erfindungsgemäßen Verfahrens ist es bevorzugt,
- in Schritt (i) den Dampf mittels eines **Apparats zur indirekten Wärmeübertragung** zu erzeugen, welcher bevorzugt ausgewählt ist aus der Gruppe bestehend aus Naturumlaufkesseln, Zwangsumlaufkesseln, Zwangsdurchlaufkesseln, Rohrbündelwärmeaustauschern, U-Rohr-Wärmeaustauschern, Doppelrohrschlangenwärmeaustauschern, Plattenwärmeaustauschern, Rohrwärmeaustauschern, Spiralwärmeaustauschern und Einsteckkondensatoren und/oder (sofern die Wärmeübertragung nicht durch direkten Dampfeintrag erfolgt)
- in Schritt (ii) die für die Durchführung des Wärme verbrauchenden Vorgangs erforderliche Wärmemenge mittels eines **Apparats zur indirekten Wärmeübertragung** bereitzustellen, welcher bevorzugt ausgewählt ist aus der Gruppe bestehend aus Naturumlaufverdampfern, Zwangsumlaufverdampfern, Einsteckverdampfern, Selbstumlaufverdampfern (z. B. sog. Robert-Verdampfer, siehe Reinhard Billet: Verdampfung und ihre technischen Anwendungen, Weinheim, 1981, S. 119 f.) Kletterverdampfern, Fallfilmverdampfern, Plattenverdampfern, Wendelrohrverdampfern, Dünnschichtverdampfern, Kurzwegverdampfern, Rohrbündelwärmeaustauschern und Spiralwärmeaustauschern.

In allen Ausführungsformen des erfindungsgemäßen Verfahrens ist es außerdem bevorzugt, durch Einstellung
- der Größe des von der Dampf abgebenden Stelle in Schritt (i) zur Position des Dampf verbrauchenden Vorgangs in Schritt (ii) geleiteten Dampfstromes oder
- der Nennweite der in Schritt (ii) eingesetzten Rohrleitung oder
- der Größe des von der Dampf abgebenden Stelle in Schritt (i) zur Position des Dampf verbrauchenden Vorgangs in Schritt (ii) geleiteten Dampfstromes und der Nennweite der Rohrleitung aus Schritt (iii)
eine aus dem Quotienten des Volumenstroms des Dampfes und dem Querschnitt der Rohrleitung berechnete **Geschwindigkeit des Dampfstromes in der Rohrleitung** im Bereich von 10,0 m/s bis 40,0 m/s zu bewirken.

Diese Vorgehensweis stellt sicher, dass das Verfahren weder infolge zu geringer Dampfgeschwindigkeiten unwirtschaftlich wird (weil bei zu geringen Dampfgeschwindigkeiten der Durchmesser der Dampfleitung größer gewählt werden muss, was wiederum erhöhten Material- und Isolierungsaufwand nach sich zieht) noch infolge zu großer Dampfgeschwindigkeiten Betriebs- oder sogar Sicherheitsprobleme auftreten (weil bei zu großen Dampfgeschwindigkeiten Erosion an der Innenwand des Dampfrohres bis hin zur Wandabtragung auftreten kann).

Die von der den Dampf abgebenden Stelle in Schritt (i) bereitgestellte Dampfmenge kann dabei in mehrere Teilströme aufgeteilt werden, von denen jeder einem anderen Wärme verbrauchenden Vorgang zugeführt wird. Der *Volumenstrom des Dampfes* kann dabei aus dem bekannten Massenstrom des Dampfes und der bei den herrschenden Bedingungen von Druck und Temperatur vorliegenden Dichte, die vom Fachmann in einfacher Weise aus ihm zugänglichen Tabellenwerken ermittelt werden kann, leicht berechnet werden. Die Einstellung der Dampfgeschwindigkeit im o. g. Bereich bewirkt, dass zu große Rohrleitungsquerschnitte auf der einen Seite und zu hohe Druckverluste sowie die Gefahr von Erosion (im Falle von Tröpfchenbildung im Dampf) auf der anderen Seite vermieden werden.

In allen Ausführungsformen der Erfindung ist es ferner bevorzugt, dass die in Schritt (ii) einzusetzende Rohrleitung eine Länge von maximal 400 m, bevorzugt von maximal 350 m und besonders bevorzugt von maximal 300 m, und einen Innendurchmesser dᵢ im Bereich von 300,0 mm bis 1000 mm aufweist. Die Mindestlänge der Rohrleitung ergibt sich aus den baulichen Gegebenheiten inkl. Sicherheitsanforderungen (z. B. ein gebotener Mindestabstand zu bestimmten Anlagenteilen) und unterliegt ansonsten keinen Beschränkungen. Es ist bevorzugt, die Länge der Rohrleitung auf das unter Berücksichtigung der baulichen Gegebenheiten ermittelte Minimum zu beschränken. Für jede gewählte Rohrleitungslänge kann ein Innendurchmesser der Rohrleitung im Bereich von 300,0 mm bis 1000 mm verwendet werden. Im Regelfall wird sich aus den üblichen Spezifikationsangaben von Rohrleitungsherstellern sofort ablesen lassen, ob ein bestimmtes Rohr einen Innendurchmesser im genannten Bereich hat. Im Zweifelsfall kann der Innendurchmesser auf einfache Weise gemessen werden. Für die Ermittlung sowohl der Länge- als auch des Innendurchmessers ist der jeweilige Wert bei 20 °C maßgeblich. Erforderlichenfalls können Rohrleitungsstücke vor ihrem Einbau in einen Produktionsverbund in einer auf 20 °C klimatisierten Halle gemessen werden. Durch Beachtung dieser Parameter wird es ermöglicht, den Druckverlust über die Rohrleitung und Wärmeverluste möglichst gering zu halten. Die optimale Kombination aus Länge und Innendurchmesser innerhalb der oben definierten Bereiche ist von den Randbedingungen (räumliche Entfernung zwischen dampfabgebender und dampfverbrauchender Stelle, Druck des Dampfes, Massenstrom des anfallenden Dampfes) abhängig und kann vom Fachmann durch Routineexperimente und/oder Computersimulationen leicht ermittelt werden. Zur weiteren Minimierung von Wärmeverlusten ist es bevorzugt, die Rohrleitung zu isolieren. Dies gilt insbesondere bei Rohrleitungslängen nahe dem erfindungsgemäß möglichen Maximum und/oder bei besonderen Verhältnissen wie der Errichtung eines Produktionsverbunds an Orten mit niedriger Jahresdurchschnittstemperatur. Grundsätzlich möglich ist auch eine Begleitbeheizung des Dampfes; da dies jedoch die Wirtschaftlichkeit des Verfahrens verschlechtern würde, ist diese Vorgehensweise im Allgemeinen nicht die der Wahl.

Durch das erfindungsgemäße Verfahren und den erfindungsgemäßen Produktionsverbund ergeben sich die mindestens die folgenden **Vorteile:**
i) Kosten für das Abkühlen ansonsten ungenutzten Dampfes entfallen;
ii) in derjenigen Produktionsanlage, welche die Restwärme nutzt, werden höherwertige Energien, wie z. B. Dampf eines Überdrucks von 6.000 mbar, eingespart.
iii) der Netto-Dampfbedarf des gesamten Produktionsverbunds wird reduziert (Reduktion der Hauptdampferzeugung für den Produktionsverbund).

Dabei können die Vorteile i) bis iii) jeweils einzeln oder vorteilhafterweise in Kombination auftreten.

Im Folgenden soll die vorliegende Erfindung anhand von Beispielen verdeutlicht werden.

### Beispiele

### Beispiele

### Allgemeine Bedingungen für die Herstellung von einem Gemisch aus Methylendiphenylendiisoyanat und Polymethylenpolyphenylenpolyisocyanat (nachfolgend summarisch MDI) mit interner Wärmeintegration des in der ersten Lösungsmittelkolonne gewonnen Dampfes - siehe auch FIG. 1

46 t/h eines Gemisches aus Methylendiphenylendiamin und Polymethylenpolyphenylenpolyamin (2, nachfolgend summarisch MDA) einer Temperatur von 115 °C werden mit 118,5 t/h Monochlorbenzol (4, MCB) einer Temperatur von 60 °C als Lösungsmittel über einen statischen Mischer (nicht gezeigt) zu einer 30%igen MDA-Lösung (20) vermischt. Phosgen wird bereitgestellt über einen Phosgengenerator und einen Phosgenverflüssiger (beide nicht gezeigt). Im Anschluss wird das Phosgen in einem Phosgenlösungstank (1030) mit MCB auf eine 55%ige Phosgenlösung (30) eingestellt. Stündlich werden 165 Tonnen 55%ige Phosgenlösung (30) einer Temperatur von -3 °C mit 46 Tonnen MDA in Form der 30%igen MDA-Lösung (20) einer Temperatur von 76 °C in einer adiabaten Reaktion, wie in EP 1 873 142 B1 beschrieben, umgesetzt. Nach der Durchmischung der beiden Rohstofflösungen im Mischaggregat (1100) wird die erhaltene Reaktionslösung (50) mit einer Temperatur von 95 °C über eine Suspensionsleitung in einen beheizten Phosgenierturm (1200) gefahren. Am Kopf des Phosgenierturmes liegen ein absoluter Druck von 2,5 bar und eine Temperatur von 105 °C vor. Der bei der Reaktion entstandene Chlorwasserstoff wird zusammen mit Spuren an Phosgen und MCB als Gasstrom (70) abgeführt. Das flüssige Reaktionsgemisch (60) wird dem Phosgenierturm entnommen und der Aufarbeitungssequenz zugeführt. Dazu wird es als Zulaufstrom in eine beheizte Entphosgenierkolonne (2100) eingeleitet. Bei einer Kopftemperatur von 136 °C und einem absoluten Druck von 2,5 bar wird über Kopf Phosgen mit Spuren MCB und Chlorwasserstoff (90) abgeführt. Phosgen wird in einer Phosgenabsorptionskolonne (nicht gezeigt) absorbiert und in den Phosgenlösungstank (1030) gefahren, und Chlorwasserstoff wird in einen Chlorwasserstoffabsorber (nicht gezeigt) und dann zur weiteren Verwendung in einen Salzsäuretank (nicht gezeigt) geleitet. Nach Abtrennung von Chlorwasserstoff und überschüssigem Phosgen aus der Isocyanat enthaltenden Reaktionslösung wird eine Isocyanat-Rohlösung (80) erhalten, die aus dem Sumpf der Entphosgenierkolonne (2100) ausgetragen und mit einer Temperatur von 175 °C in eine erste Destillationsstufe (2200) gefahren wird, um sie vom Lösungsmittel MCB zu befreien. Der absolute Druck am Kopf dieser Lösungsmitteldestillationskolonne beträgt 800 mbar bei einer Sumpftemperatur von 155 °C. Über Kopf wird MCB gasförmig (110) bei 124 °C abgezogen und mit einem Kondensator (2310) auf 119 °C verflüssigt. Der Kondensator (2310) wird mit Kondensat gespeist, wobei 20 t/h an Dampf eines Drucks von 1,51 bar_{(abs.)} mit einer Temperatur von 112 °C anfallen. 1 t/h dieses Dampfes wird benutzt, um im Chlorwasserstoffabsorber (nicht gezeigt) Spuren an MCB aus der Salzsäure auszutreiben. Weitere 1 t/h dieses Dampfes wird für Begleitbeheizungen in der MDI-Anlage aufgewendet. Die noch verbliebenen 18 t/h dieses Dampfes mit 1,51 bar_{(abs.)} Druck werden über einen Luftkühler "vernichtet", und das verbliebene Kondensat wird dem Kreislauf des Kondensatsystems zugeführt. Das Reaktionsprodukt (100) wird aus dem Sumpf der Kolonne ausgetragen und in einer zweiten Kolonne (nicht gezeigt) bis auf 1 % von restlichem MCB befreit. Anschließend wird in einem Gegenstromverdampfer bei einem absoluten Druck von 8 mbar und einer Sumpftemperatur von 214 °C das Produkt von Nebenkomponenten wie Phenylisocyanat und restlichem MCB befreit. Dabei fallen als Sumpfprodukt der zweiten (nicht gezeigten) Kolonne 58 t/h MDI an, das mittels weiterer Destillation (2410 / 2400) aufgetrennt bzw. gereinigt wird, wobei polymere Fraktionen 141 (die auch Anteile von monomerem MDI, mMDI, enthalten) und monomere Fraktionen 140 erhalten werden, die anschließend beide zur weiteren Verwendung in die entsprechenden MDI-Produkttanks gefahren werden.

### Allgemeine Bedingungen für die Herstellung von Methylendiphenylendiamin und Polymethylenpolyphenylenpolyamin (nachfolgend summarisch MDA) - siehe auch FIG. 2

In einem kontinuierlichen Reaktionsprozess werden 76 t/h Einsatzanilin (5) mit einer Temperatur von 30 °C (enthaltend 95 Massen % Anilin) und 23,3 t/h 37%ige wässrige Formalinlösung (6) mit einer Temperatur von 40 °C vermischt und bei 93 °C und einem absoluten Druck von 1,04 bar in einem gerührten Reaktionskessel (3000) zum Aminal umgesetzt. Das den Reaktionskessel verlassende Reaktionsgemisch (7) wird in einen Phasentrennapparat (4000, "Aminalabscheider") geführt. Nach der Phasentrennung zur Entfernung der wässrigen Phase (8), die zu einem Abwassersammelbehälter (11000-1) geführt wird, wird die organische Phase (9) in einer Mischdüse (nicht gezeigt) mit 9,4 t/h an 30%iger wässriger Salzsäure (3) versetzt und in den ersten Umlagerungsreaktor gefahren. Die Umlagerungsreaktion wird in einer Reaktorkaskade (5000) bei 50 °C bis 130 °C durchgeführt. Nach vollständiger Reaktion wird das erhaltene Reaktionsgemisch (11) mit 10,7 t/h an 32%iger Natronlauge (32) versetzt und in einem Neutralisationsrührbehälter (6000) umgesetzt. Die Temperatur beträgt dabei 105 °C und der absolute Druck liegt bei 1,04 bar. Die neutralisierte Reaktionsmischung (12) wird anschließend in einem Neutralisationsabscheider (7000) in eine wässrige, untere Phase (14), die zu einem Abwassersammelbehälter (11000-1) geführt wird, und in eine organische Phase (13) getrennt. Die organische, obere Phase wird zur Wäsche geleitet und in einem gerührten Waschbehälter (8000) mit Kondensat und/oder Wasser aus internen Wasserkreisläufen (Anilin/Wassergemische) (15) gewaschen. Nach Abtrennung des Waschwassers (18) in einem Waschwasserabscheider (9000) wird das so erhaltene Roh-MDA (17) mit 8,6 t/h an 6 bar (ü) Dampf in (10000-1) vorverdampft und mit Hilfe von 2 t/h 6 bar (ü) Strippdampf in der Destillationsapparatur (10000-2) von Wasser und Anilin (22) befreit, wobei als Sumpfprodukt (21) 50 t/h MDA anfallen. Das Waschwasser (18) und die kondensierten Kopfprodukte der Destillationsapparatur (10000-1) - Strom (19) enthaltend Anilin und Wasser- und der Destillationsapparatur (10000-2) - Strom (22) enthaltend Anilin und Wasser -, werden zu einem Abwassersammelbehälter (11000-1) geführt. Das gesammelte Abwasser wird in einem Trennbehälter (11000-3) in eine organische Phase (24), enthaltend (feuchtes, mit Wasser bis zur Löslichkeitsgrenze gesättigtes) Anilin, und eine wässrige Phase (25), enthaltend organische Bestandteile, getrennt. Die wässrige Phase (25) wird in einem Abwasserstripper (11000-5) mit Hilfe von 13 t/h 6 bar (ü) Strippdampf von Anilin (29) befreit, bevor es in die Abwasserendbehandlung geht.

### Beispiel 1 (erfindungsgemäß): Nutzung der Restenergie aus der Wärmeintegration der ersten Lösungsmittelkolonne des MDI als 1,51 bar_{(abs.)}-Dampf im MDA-Prozess

Die Herstellung von 58 t/h MDI in kontinuierlicher Fahrweise wird, wie in den allgemeinen Bedingungen beschrieben, durchgeführt. Die Restwärme aus der Wärmeintegration der Lösungsmitteldestillationskolonne der MDI-Anlage wird als Dampf eines Drucks von 1,51 bar_{(abs.)} mit einer Temperatur von 112 °C über eine Rohrleitung DN700 (Innendurchmesser 695,0 mm) in die **MDA**-Anlage geschickt. Die Rohrleitungslänge beträgt zwischen der Lösungsmitteldestillationskolonne der MDI-Anlage und der Verzweigung in der MDA-Anlage 235 m. 8 t/h dieses Dampfes werden in der MDA-Anlage zur Beheizung der Vorverdampfung (10000-1) des Einlaufstromes zu der MDA-Kolonne benutzt (weitere 25 m Rohrleitung DN300; Innendurchmesser 309,7 mm), weitere 2 t/h dieses Dampfes werden als Strippdampf in der MDA-Kolonne (10000-2) eingesetzt (weitere 15 m Rohrleitung), und die verbleibenden 8 t/h dieses Dampfes werden im Abwasserstripper (11000-5) verwendet (weitere 40 m Rohrleitung DN300). Die noch fehlende Menge von 4,1 t/h an 1,51 bar_{(abs}.₎-Dampf für den kompletten Ersatz des ansonsten für den Betrieb des Abwasserstrippers (11000-5) benötigten 7,01 bar_{(abs.)}-Dampfes wird aus einer naheliegenden Anilinanlage der MDI-Verfahrenskette geliefert.

**Fazit:** Mit der erfindungsgemäßen Verfahrensweise werden insgesamt 19,2 t/h Dampf eines Drucks von 7,01 bar_{(abs.)} in der MDA-Anlage eingespart.

### Beispiel 2 (erfindungsgemäß): Nutzung der Restenergie aus der Wärmeintegration der ersten Lösungsmittelkolonne des MDI als 1,51 bar_{(abs.)}-Dampf im MDA-Prozess

Die Herstellung von 58 t/h MDI in kontinuierlicher Fahrweise wird, wie in den allgemeinen Bedingungen beschrieben, durchgeführt. Die erste Lösungsmitteldestillationskolonne (2200) wird dabei mit anderen Parametern betrieben, wobei ein höherwertiger Dampf entsteht. Wie in den allgemeinen Bedingungen beschrieben, wird die Isocyanat-Rohlösung aus dem Sumpf der Entphosgenierkolonne (2100) ausgetragen und mit einer Temperatur von 177 °C in die erste Destillationsstufe (2200) gefahren. Der absolute Druck am Kopf dieser Lösungsmitteldestillationskolonne beträgt 1,5 bar bei einer Sumpftemperatur von 155 °C. Über Kopf wird MCB (110) gasförmig bei 147 °C abgezogen und mit einem Kondensator (2310) auf 140 °C verflüssigt. Der Kondensator (2310) wird mit Kondensat gespeist, wobei 19,6 t/h an Dampf eines Drucks von 1,51 bar_{(abs.)} mit einer Temperatur von 112 °C anfallen. 0,8 t/h dieses Dampfes werden benutzt, um im Chlorwasserstoffabsorber (nicht gezeigt) Spuren an MCB aus der Salzsäure auszutreiben. Weitere 0,8 t/h dieses Dampfes werden für Begleitbeheizungen in der MDI-Anlage aufgewendet. Die noch verbliebenen 18 t/h dieses 1,51 bar_{(abs.)}-Dampfes der Restwärme aus der Wärmeintegration der Lösungsmitteldestillationskolonnen der MDI-Anlage wird als 1,51 bar_{(abs.)}-Dampf mit einer Temperatur von 112 °C über eine Verteilerrohrleitung in die **MDA**-Anlage geschickt. 2 t/h dieses Dampfes werden als Strippdampf in der MDA-Kolonne (10000-2) eingesetzt, weitere 12,1 t/h dieses Dampfes werden im Abwasserstripper (11000-5) verwendet, und die restlichen Dampfmengen werden in einer **Nitrobenzol**anlage der MDI-Verfahrenskette eingesetzt.

**Fazit:** Mit der erfindungsgemäßen Verfahrensweise werden 14 t/h Dampf eines Drucks von 7,01 bar_{(abs.)} in der MDA-Anlage gespart, und wegen des höheren Dampfdruckes des Restwärmedampfes im Vergleich zu Beispiel 1 ist der Rohrleitungsdurchmesser der Wärmetransportleitung zwischen den Anlagen 33 % kleiner, wodurch die Investition in die Rohrleitung geringer ausfällt und auch geringere Wärmeverluste zu verzeichnen sind.

### Beispiel 3 (erfindungsgemäß): Nutzung der Restenergie aus der Wärmeintegration der ersten Lösungsmittelkolonne des MDI als 1,51 bar_{(abs.)}-Dampf im MDA-Prozess

Es wird verfahren wie in Beispiel 2 mit dem Unterschied, dass die restlichen Dampfmengen nicht wie in Beispiel 2 in einer Nitrobenzolanlage der MDI-Verfahrenskette, sondern stattdessen in der Chlorwasserstoffabsorption der TDI-Anlage einer benachbarten TDI-Verfahrenskette eingesetzt werden.

## Patentansprüche

1. Verfahren zur Herstellung von *m* Isocyanaten in *m* Verfahrensketten, in denen jeweils ein Isocyanat als Endprodukt über mindestens ein Zwischenprodukt hergestellt wird,
wobei jede der *m* Verfahrensketten in mindestens 2 Produktionsanlagen (100000-1, 100 000-2, ...) durchgeführt wird, nämlich in einer ersten Produktionsanlage (100 000-1) ausgelegt für die Herstellung des mindestens einen Zwischenprodukts und in einer zweiten Produktionsanlage (100 000-2) ausgelegt für die Herstellung des Isocyanat-Endprodukts, wobei die mindestens 2 Produktionsanlagen (100 000-1, 100 000-2, ...,) Bestandteil eines Produktionsverbunds (1 000 000) sind,
wobei in dem Produktionsverbund (1 000 000) insgesamt *n* chemische Produkte, die ausgewählt sind aus der Gruppe bestehend aus Zwischenprodukten, Katalysatoren und Isocyanat-Endprodukten, hergestellt werden, wobei *n* eine natürliche Zahl im Bereich von 2 bis 40, bevorzugt im Bereich von 2 bis 20, besonders bevorzugt im Bereich von 2 bis 15, ist und wobei *m* eine natürliche Zahl im Bereich von 1 bis *n* ist,
wobei bei der Herstellung jedes der *n* chemischen Produkte mindestens ein Wärme abgebender oder Wärme verbrauchender Vorgang stattfindet und im Verfahren zur Herstellung der *m* Isocyanate insgesamt mindestens ein Wärme abgebender und mindestens ein Wärme verbrauchender Vorgang stattfinden,
wobei
(i) die in einem Wärme abgebenden Vorgang der Herstellung eines der chemischen Produkte in einer ersten Produktionsanlage (100 000-1) freiwerdende Wärmeenergie mindestens teilweise zur Erzeugung von Dampf, insbesondere von Wasserdampf, eines Drucks von 1,31 bar_{(abs.)} bis 1,91 bar_{(abs.)} und einer Temperatur von 107 °C bis 119 °C genutzt wird;
(ii) der in (i) erzeugte Dampf für die Durchführung eines Wärme verbrauchenden Vorgangs in der Herstellung eines von dem in Schritt (i) hergestellten chemischen Produkt verschiedenen chemischen Produktes eingesetzt wird, wobei die Herstellung dieses von dem in Schritt (i) hergestellten chemischen Produkt verschiedenen chemischen Produktes in einer zweiten Produktionsanlage (100000-2) stattfindet, wobei der in (i) erzeugte Dampf von der Dampf abgebenden Stelle in Schritt (i) über eine Rohrleitung zur Position des Wärme verbrauchenden Vorgangs in Schritt (ii) geleitet wird.

2. Verfahren gemäß Anspruch 1, bei welchem das Isocyanat-Endprodukt ausgewählt ist aus der Gruppe bestehend aus Methylendiphenylendiisocyanat, Polymethylenpolyphenylenpolyisocyanat, Gemischen aus Methylendiphenylendiisocyanat und Polymethylenpolyphenylenpolyisocyanat, Toluylendiisocyanat, Xylylendiisocyanat, 1,5-Pentandiisocyanat, Hexamethylendiisocyanat, Isophorondiisocyanat und Naphthyldiisocyanat.

3. Verfahren gemäß Anspruch 1 oder 2, bei welchem das Isocyanat-Endprodukt ein aromatisches Isocyanat ist und bei welchem
in einer ersten Verfahrenskette (α)
A) in einer ersten Verfahrensstufe ein aromatischer Kohlenwasserstoff, insbesondere Toluol, zu einer aromatischen Nitroverbindung, insbesondere Dinitrotoluol, nitriert wird;
B) die aromatische Nitroverbindung, insbesondere Dinitrotoluol, in einer zweiten Verfahrensstufe durch Hydrierung zu einem aromatischen Amin, insbesondere Toluylendiamin, hydriert wird;
C) das aromatische Amin, insbesondere Toluylendiamin, in einer dritten Verfahrensstufe durch Phosgenierung zum aromatischen Isocyanat-Endprodukt, insbesondere Toluylendiisocyanat, umgesetzt wird;
und/oder
in einer zweiten Verfahrenskette (β)
A) in einer ersten Verfahrensstufe ein aromatischer Kohlenwasserstoff, insbesondere Benzol, zu einer aromatischen Nitroverbindung, insbesondere Nitrobenzol, nitriert wird;
B) die aromatische Nitroverbindung, insbesondere Nitrobenzol, in einer zweiten Verfahrensstufe durch Hydrierung zu einem aromatischen Amin, insbesondere Anilin, hydriert wird;
C) das aromatische Amin, insbesondere Anilin, in einer dritten Verfahrensstufe zu einem anderen aromatischen Amin, insbesondere Methyl endiphenylendiamin, Polymethylenpolyphenylenpolyamin und/oder Gemischen aus Methylendiphenylendiamin und Polymethylenpolyphenylenpolyamin, umgesetzt wird;
D) das aromatische Amin, insbesondere Methylendiphenyldiamin, Polymethylenpolyphenylenpolyamin und/oder Gemische aus Methylendiphenyldiamin und Polymethylenpolyphenylenpolyamin, in einer vierten Verfahrensstufe durch Phosgenierung zum aromatischen Isocyanat-Endprodukt, insbesondere Methylendiphenyldiisocyanat, Polymethylenpolyphenylenpolyisocyanat und/oder Gemischen aus Methylendiphenyldiisocyanat und Polymethylenpolyphenylenpolyisocyanat, umgesetzt wird.

4. Verfahren gemäß Anspruch 3, bei welchem die Phosgenierung gemäß Verfahrensstufe (α)C) und/oder gemäß Verfahrensstufe (β) D) Folgendes umfasst:
I) Umsetzung des zu phosgenierenden Amins (2) mit Phosgen (3) in der Flüssigphase und Trennung des erhaltenen Verfahrensproduktes in einen flüssigen, das Roh-Isocyanat und Lösungsmittel enthaltenden Strom (60) und einen gasförmigen, Phosgen und Chlorwasserstoff enthaltenden Strom (70);
II) Trennung des flüssigen Stroms (60) aus Schritt I) in einen flüssigen, Lösungsmittel und Roh-Isocyanat enthaltenden Strom (80) und einen gasförmigen, Phosgen und Chlorwasserstoff enthaltenden Strom (90) in einer Destillationsvorrichtung (2100);
III) Trennung des flüssigen Stroms (80) in einen gasförmigen, Lösungsmittel enthaltenden Strom (110) und einen flüssigen, Roh-Isocyanat enthaltenden Strom (100) in einer Destillationsvorrichtung (2200);
IV) Trennung des gasförmigen Stroms (110) nach dessen mindestens teilweiser Verflüssigung in einem Kondensator (2310), in einen flüssigen, Lösungsmittel enthaltenden Strom (120) und einen gasförmigen, Phosgen enthaltenden Strom (130) in einer Destillationsvorrichtung (2300);
V) Gewinnung eines flüssigen Isocyanat-Stroms (140) aus dem flüssigen Strom (100), wobei ein Nebenkomponenten und ggf. Lösungsmittel enthaltender gasförmiger Strom (150) anfällt, in einer Destillationsvorrichtung (2400), optional umfassend einen Schritt V.1), die Abtrennung von polymeren Isocyanatfraktionen in einer vorgeschalteten Einrichtung zur Polymerabtrennung (2410) als Strom (141).

5. Verfahren gemäß Anspruch 4, umfassend die Verfahrenskette (β), wobei das in der Verfahrensstufe (β) D) zu phosgenierende Amin ein Gemisch aus Methylendiphenylendiamin und Polymethylenpolyphenylenpolyamin ist, wobei ferner Schritt V.1) durchgeführt wird und so das Isocyanat-Endprodukt in den zwei Strömen 140 und 141 anfällt, wobei Strom 140 Methylendiphenylendiisocyanat und Strom 141 ein Gemisch aus Methylendiphenylendiisocyanat und Polymethylenpolyphenylenpolyisocyanat umfasst.

6. Verfahren gemäß Anspruch 5, bei welchem der Wärme abgebende Vorgang aus Schritt (i) die mindestens teilweise Verflüssigung des gasförmigen Stroms (110) im Kondensator (2310) ist und bei welchem der durch die Wärmeabgabe erzeugte Dampf in die Verfahrensstufe (β) C) geführt wird.

7. Verfahren gemäß Anspruch 6, bei welchem die Verfahrensstufe (β) C) die folgenden Schritte umfasst:
IA) Reaktion von Anilin und Formaldehyd in einem Reaktor (3000) in Abwesenheit eines sauren Katalysators unter Bildung eines Aminals, gefolgt von Trennung des erhaltenen Reaktionsgemisches in eine wässrige und eine organische, das Aminal enthaltende Phase in einer in den Reaktor integrierten Phasentrenneinrichtung oder in einem separaten Phasentrennapparat (4000);
IIA) Reaktion zumindest eines Teils der in Schritt IA) erhaltenen organischen, das Aminal enthaltenden Phase in einem Reaktor (5000) mit Säure, wobei das Aminal zu einem Gemisch aus Methylendiphenylendiamin und Polymethylenpolyphenylenpolyamin reagiert;
oder
IB) Reaktion von Anilin und Säure in einem Reaktor;
IIB) Reaktion zumindest eines Teils des in Schritt IB) erhaltenen Reaktionsgemisches in einem Reaktor mit Formaldehyd zu einem Gemischen aus Methylendiphenylendiamin und Polymethylenpolyphenylenpolyamin;
III) Neutralisation des in Schritt IIA) oder IIB) erhaltenen Reaktionsgemisches in einem Reaktor (6000);
IV) Auftrennen des in Schritt III) erhaltenen neutralisierten Reaktionsgemisches in eine organische Phase, umfassend ein Gemisch aus Methylendiphenylendiamin und Polymethylenpolyphenylenpolyamin, und in eine wässrige Phase in einem Trennbehälter (7000);
V) Waschen der organischen Phase mit Waschflüssigkeit in einem Waschbehälter (8000);
VI) Auftrennen des in Schritt V) erhaltenen Gemisches in eine organische Phase, umfassend ein Gemisch aus Methylendiphenylendiamin und Polymethylenpolyphenylenpolyamin, und in eine wässrige Phase in einem Trennbehälter (7000);
VII) Destillieren der organischen Phase aus Schritt VI) in einer Destillationsapparatur (10000) umfassend eine erste Destillationsstufe (10000-1) zur Abtrennung eines Wasser und Anilin enthaltenden Stromes und eine zweite Destillationsstufe (10000-2) zur weiteren Reinigung des in der ersten Stufe nach Abtrennung des Wasser und Anilin enthaltenden Stromes verbleibenden Verfahrensprodukts unter Erhalt eines Gemisches aus Methylendiphenylendiamin und Polymethylenpolyphenylenpolyamin, wobei in der zweiten Stufe (10000-2) optional Strippdampf zur direkten Wärmeübertragung eingesetzt wird;
VIII) optionale Abtrennung von Methylendiphenylendiamin aus dem in Schritt VII) erhaltenen Gemisch aus Methylendiphenylendiamin und Polymethylenpolyphenylenpolyamin.

8. Verfahren gemäß Anspruch 7, bei welchem bei welchem die Verfahrensstufe (β) C) zusätzlich Folgendes umfasst:
IX) Aufarbeitung
• der wässrigen Phase aus Schritt IA) oder
• der wässrigen Phase aus Schritt IV) oder
• der wässrigen Phase aus Schritt VI) oder
• des Wasser und Anilin enthaltenden Stroms aus Schritt VII) oder
• einer Mischung von zwei oder mehr der vorgenannten wässrigen Verfahrensprodukte
in einer Abwasseraufarbeitungseinrichtung (11000) umfassend einen Abwassersammelbehälter (11000-1), optional einen Abwassererhitzer (11000-2), einen Trennbehälter (11000-3), optional einen Extraktionsbehälter (11000-4) und eine Abwasserdestillationskolonne (11000-5);
wobei der wärmeverbrauchende Vorgang aus Schritt (ii) ausgewählt ist aus der Gruppe bestehend aus:
• der Abtrennung des Wasser und Anilin enthaltenden Stromes aus der organischen Phase aus Schritt VI) in der Destillationsstufe (10000-1),
• der weiteren Reinigung des in der ersten Destillationsstufe (10000-1) nach Abtrennung des Wasser und Anilin enthaltenden Stromes verbleibenden Verfahrensprodukts in der Destillationsstufe (10000-2), wobei bei Einsatz von Strippdampf zur direkten Wärmeübertragung in (10000-2) dieser Strippdampf den in Schritt (i) erzeugten Dampf umfasst und bevorzugt nur aus dem in Schritt (i) erzeugten Dampf besteht,
• sofern der Abwassererhitzer (11000-2) vorhanden ist, der Erwärmung dieses Abwassererhitzers (11000-2) und
• Kombinationen von zwei oder mehr der vorgenannten Vorgänge.

9. Verfahren gemäß Anspruch 7 oder 8, welches zusätzlich die Verfahrensstufe der Herstellung von Salzsäure als ein weiteres chemisches Produkt umfasst, wobei die Salzsäure durch Absorption von Chlorwasserstoff in Wasser oder 0,1%iger bis 20%iger Salzsäure in einer Absorptionskolonne hergestellt wird, wobei die so hergestellte Salzsäure als Säure in Schritt (β) C) IIA) oder (β) C) IB) eingesetzt wird,
wobei der Wärme verbrauchende Vorgang das direkte oder indirekte Beheizen des Sumpfes der Absorptionskolonne aus der Verfahrensstufe der Herstellung von Salzsäure ist.

10. Verfahren gemäß Anspruch 3, bei welchem die Phosgenierung in Verfahrensstufe (α) C) und/oder in Verfahrensstufe (β) D) Folgendes umfasst:
0) Bereitstellung gasförmigen Phosgens (3) durch Verdampfung flüssigen Phosgens;
I) Verdampfung des zu phosgenierenden Amins (2), Umsetzung des verdampften Amins mit gasförmigem Phosgen (3) aus Schritt 0) in der Gasphase, Abbruch der Umsetzung durch Inkontaktbringen mit einem Lösungsmittel-haltigen Strom einer Temperatur unterhalb des Siedepunkts des Amins und Trennung des erhaltenen Verfahrensproduktes in einen flüssigen, das Roh-Isocyanat und Lösungsmittel enthaltenden Strom (60) und einen gasförmigen, Phosgen und Chlorwasserstoff enthaltenden Strom (70);
II) Trennung des flüssigen Stroms (60) aus Schritt I) in einen flüssigen, Lösungsmittel und Roh-Isocyanat enthaltenden Strom (80) und einen gasförmigen, Phosgen und Chlorwasserstoff enthaltenden Strom (90) in einer Destillationsvorrichtung (2100);
III) Trennung des flüssigen Stroms (80) in einen gasförmigen, Lösungsmittel enthaltenden Strom (110) und einen flüssigen, Roh-Isocyanat enthaltenden Strom (100) in einer Destillationsvorrichtung (2200);
IV) Trennung des gasförmigen Stroms (110) nach dessen mindestens teilweiser Verflüssigung in einem Kondensator (2310), in einen flüssigen, Lösungsmittel enthaltenden Strom (120) und einen gasförmigen, Phosgen enthaltenden Strom (130) in einer Destillationsvorrichtung (2300);
V) Gewinnung eines flüssigen Isocyanat-Stroms (140) aus dem flüssigen Strom (100), wobei ein Nebenkomponenten und ggf. Lösungsmittel enthaltender gasförmiger Strom (150) und ein Gemisch aus polymeren Anteilen und monomerem Isocyanat als Strom (143) anfallen, in einer bevorzugt als Trennwandkolonne ausgelegten Destillationsvorrichtung (2400).

11. Verfahren gemäß Anspruch 10, umfassend die Verfahrenskette (α), bei welchem der Wärme verbrauchende Vorgang aus Schritt (ii) mindestens ein Teilschritt der Verdampfung des flüssigen Phosgens in Schritt 0) ist und bei welchem der Wärme abgebende Vorgang aus Schritt (i) aus der Verfahrensstufe (α) B) stammt.

12. Verfahren gemäß Anspruch 3, umfassend Verfahrenskette (α) und Verfahrenskette (β), bei welchem
der mindestens eine Wärme abgebende Vorgang aus Schritt (i) Bestandteil der Verfahrenskette (α) und der mindestens eine Wärme verbrauchende Vorgang aus Schritt (ii) Bestandteil der Verfahrenskette (β) ist
oder bei welchem
der mindestens eine Wärme abgebende Vorgang aus Schritt (i) Bestandteil der Verfahrenskette (β) und der mindestens eine Wärme verbrauchende Vorgang aus Schritt (ii) Bestandteil der Verfahrenskette (α) ist.

13. Verfahren gemäß einem der vorstehenden Ansprüche, bei welchem
• in Schritt (i) der Dampf mittels eines Apparats zur indirekten Wärmeübertragung erzeugt wird, welcher bevorzugt ausgewählt ist aus der Gruppe bestehend aus Naturumlaufkesseln, Zwangsumlaufkesseln, Zwangsdurchlaufkesseln, Rohrbündelwärmeaustauschern, U-Rohr-Wärmeaustauschern, Doppelrohrschlangenwärmeaustauschern, Plattenwärmeaustauschern, Rohrwärmeaustauschern, Spiralwärmeaustauschern und Einsteckkondensatoren oder
• in Schritt (ii) die für die Durchführung des Wärme verbrauchenden Vorgangs erforderliche Wärmemenge mittels eines Apparats zur indirekten Wärmeübertragung bereitgestellt wird, welcher bevorzugt ausgewählt ist aus der Gruppe bestehend aus Naturumlaufverdampfern, Zwangsumlaufverdampfern, Einsteckverdampfern, Selbstumlaufverdampfern, Kletterverdampfern, Fallfilmverdampfern, Plattenverdampfern, Wendelrohrverdampfern, Dünnschichtverdampfern, Kurzwegverdampfern, Rohrbündelwärmeaustauschern und Spiralwärmeaustauschern oder
• in Schritt (i) der Dampf mittels eines Apparats zur indirekten Wärmeübertragung erzeugt wird, welcher bevorzugt ausgewählt ist aus der Gruppe bestehend aus Naturumlaufkesseln, Zwangsumlaufkesseln, Zwangsdurchlaufkesseln, Rohrbündelwärmeaustauschern, U-Rohr-Wärmeaustauschern, Doppelrohrschlangenwärmeaustauschern, Plattenwärmeaustauschern, Rohrwärmeaustauschern, Spiralwärmeaustauschern und Einsteckkondensatoren und in Schritt (ii) die für die Durchführung des Wärme verbrauchenden Vorgangs erforderliche Wärmemenge mittels eines Apparats zur indirekten Wärmeübertragung bereitgestellt wird, welcher bevorzugt ausgewählt ist aus der Gruppe bestehend aus Naturumlaufverdampfern, Zwangsumlaufverdampfern, Einsteckverdampfern, Selbstumlaufverdampfern, Kletterverdampfern, Fallfilmverdampfern, Plattenverdampfern, Wendelrohrverdampfern, Dünnschichtverdampfern, Kurzwegverdampfern, Rohrbündelwärmeaustauschern und Spiralwärmeaustauschern.

14. Verfahren gemäß einem der vorstehenden Ansprüche, bei welchem durch Einstellung
• der Größe des von der Dampf abgebenden Stelle in Schritt (i) zur Position des Wärme verbrauchenden Vorgangs in Schritt (ii) geleiteten Dampfstromes oder
• der Nennweite der in Schritt (ii) eingesetzten Rohrleitung oder
• der Größe des von der Dampf abgebenden Stelle in Schritt (i) zur Position des Wärme verbrauchenden Vorgangs in Schritt (ii) geleiteten Dampfstromes und der Nennweite der Rohrleitung aus Schritt (iii)
eine aus dem Quotienten des Volumenstroms des Dampfes und dem Querschnitt der Rohrleitung berechnete Geschwindigkeit des Dampfstromes in der Rohrleitung im Bereich von 10,0 m/s bis 40,0 m/s bewirkt wird.

15. Verfahren gemäß einem der vorstehenden Ansprüche, bei welchem die Rohrleitung eine Länge von maximal 400 m und einem Innendurchmesser dᵢ im Bereich von 300,0 mm bis 1000 mm aufweist.

## Claims

1. Process for preparing m isocyanates in m process chains, in each of which an isocyanate is prepared as end product via at least one intermediate, wherein each of the m process chains is conducted in at least 2 production plants (100 000-1, 100 000-2, ...), namely in a first production plant (100 000-1) designed for the preparation of the at least one intermediate and in a second production plant (100 000-2) designed for the preparation of the isocyanate end product, wherein the at least 2 production plants (100 000-1, 100 000-2, ...,) are part of an integrated production system (1 000 000), wherein a total of *n* chemical products selected from the group consisting of intermediates, catalysts and isocyanate end products are prepared in the integrated production system (1 000 000), where *n* is a natural number in the range from 2 to 40, preferably in the range from 2 to 20, more preferably in the range from 2 to 15, and where m is a natural number in the range from 1 to *n*, wherein, in the preparation of each of the *n* chemical products, at least one heat-releasing or heat-consuming operation takes place and, in the process for preparing the *m* isocyanates, a total of at least one heat-releasing operation and at least one heat-consuming operation take place,
wherein
(i) the heat energy released in a heat-releasing operation in the preparation of one of the chemical products in a first production plant (100 000-1) is utilized at least partly for generation of vapor, especially of steam, at a pressure of 1.31 bar_{(abs.)} to 1.91 bar_{(abs.)} and a temperature of 107°C to 119°C;
(ii) the vapor generated in (i) is used for the performance of a heat-consuming operation in the preparation of a chemical product different than the chemical product prepared in step (i), wherein the preparation of this chemical product different than the chemical product prepared in step (i) takes place in a second production plant (100 000-2), wherein the vapor generated in (i) is guided from the vapor-releasing point in step (i) via a pipeline to the position of the heat-consuming operation in step (ii).

2. Process according to Claim 1, in which the isocyanate end product is selected from the group consisting of methylene diphenylene diisocyanate, polymethylene polyphenylene polyisocyanate, mixtures of methylene diphenylene diisocyanate and polymethylene polyphenylene polyisocyanate, tolylene diisocyanate, xylylene diisocyanate, pentane 1,5-diisocyanate, hexamethylene diisocyanate, isophorone diisocyanate and naphthyl diisocyanate.

3. Process according to Claim 1 or 2, in which the isocyanate end product is an aromatic isocyanate and in which
in a first process chain (α)
A) in a first process stage an aromatic hydrocarbon, especially toluene, is nitrated to give an aromatic nitro compound, especially dinitrotoluene;
B) the aromatic nitro compound, especially dinitrotoluene, is hydrogenated in a second process stage by hydrogenation to give an aromatic amine, especially tolylenediamine;
C) the aromatic amine, especially tolylenediamine, is converted in a third process stage by phosgenation to give the aromatic isocyanate end product, especially tolylene diisocyanate;
and/or
in a second process chain (β)
A) in a first process stage an aromatic hydrocarbon, especially benzene, is nitrated to give an aromatic nitro compound, especially nitrobenzene;
B) the aromatic nitro compound, especially nitrobenzene, is hydrogenated in a second process stage by hydrogenation to give an aromatic amine, especially aniline;
C) the aromatic amine, especially aniline, is converted in a third process stage to another aromatic amine, especially methylene diphenylene diamine, polymethylene polyphenylene polyamine and/or mixtures of methylene diphenylene diamine and polymethylene polyphenylene polyamine;
D) the aromatic amine, especially methylene diphenyl diamine, polymethylene polyphenylene polyamine and/or mixtures of methylene diphenyl diamine and polymethylene polyphenylene polyamine, is converted in a fourth process stage by phosgenation to give the aromatic isocyanate end product, especially methylene diphenyl diisocyanate, polymethylene polyphenylene polyisocyanate and/or mixtures of methylene diphenyl diisocyanate and polymethylene polyphenylene polyisocyanate.

4. Process according to Claim 3, in which the phosgenation in process stage (α) C) and/or in process stage (β) D) comprises the following:
I) reacting the amine (2) to be phosgenated with phosgene (3) in the liquid phase and separating the process product obtained into a liquid stream (60) comprising the crude isocyanate and solvent, and a gaseous stream (70) comprising phosgene and hydrogen chloride;
II) separating the liquid stream (60) from step I) into a liquid stream (80) comprising solvent and crude isocyanate, and a gaseous stream (90) comprising phosgene and hydrogen chloride in a distillation apparatus (2100);
III) separating the liquid stream (80) into a gaseous stream (110) comprising solvent and a liquid stream (100) comprising crude isocyanate in a distillation apparatus (2200);
IV) separating the gaseous stream (110), after it has been at least partly liquefied in a condenser (2310), into a liquid stream (120) comprising solvent and a gaseous stream (130) comprising phosgene in a distillation apparatus (2300) ;
V) obtaining a liquid isocyanate stream (140) from the liquid stream (100), resulting in a gaseous stream (150) comprising secondary components and optionally solvent, in a distillation apparatus (2400), optionally comprising a step V.1), the removal of polymeric isocyanate fractions in an upstream unit for polymer removal (2410) as stream (141).

5. Process according to Claim 4, comprising process chain (β), wherein the amine to be phosgenated in process stage (β) D) is a mixture of methylene diphenylene diamine and polymethylene polyphenylene polyamine, wherein step V.1) is also conducted and hence the isocyanate end product is obtained in the two streams 140 and 141, wherein stream 140 comprises methylene diphenylene diisocyanate and stream 141 a mixture of methylene diphenylene diisocyanate and polymethylene polyphenylene polyisocyanate.

6. Process according to Claim 5, in which the heat-releasing operation from step (i) is the at least partial liquefaction of the gaseous stream (110) in the condenser (2310), and in which the vapor generated by the release of heat is guided into process stage (β) C).

7. Process according to Claim 6, in which process stage (β) C) comprises the following steps:
IA) reacting aniline and formaldehyde in a reactor (3000) in the absence of an acidic catalyst to form an aminal, followed by separating the reaction mixture obtained into an aqueous phase and an organic phase comprising the aminal in a phase separation unit integrated into the reactor or in a separate phase separation apparatus (4000);
IIA) reacting at least a portion of the organic phase comprising the aminal which is obtained in step IA) in a reactor (5000) with acid, with reaction of the aminal to give a mixture of methylene diphenylene diamine and polymethylene polyphenylene polyamine;
or
IB) reacting aniline and acid in a reactor;
IIB) reacting at least a portion of the reaction mixture obtained in step IB) in a reactor with formaldehyde to give a mixture of methylene diphenylene diamine and polymethylene polyphenylene polyamine;
III) neutralizing the reaction mixture obtained in step IIA) or IIB) in a reactor (6000);
IV) separating the neutralized reaction mixture obtained in step III) into an organic phase comprising a mixture of methylene diphenylene diamine and polymethylene polyphenylene polyamine and into an aqueous phase in a separation vessel (7000);
V) washing the organic phase with washing liquid in a wash vessel (8000);
VI) separating the mixture obtained in step V) into an organic phase comprising a mixture of methylene diphenylene diamine and polymethylene polyphenylene polyamine and into an aqueous phase in a separation vessel (7000);
VII) distilling the organic phase from step VI) in a distillation apparatus (10000) comprising a first distillation stage (10000-1) for removal of a water- and aniline-containing stream and a second distillation stage (10000-2) for further purification of the process product remaining in the first stage after removal of the water- and aniline-containing stream to obtain a mixture of methylene diphenylene diamine and polymethylene polyphenylene polyamine, with optional use of stripping vapor in the second stage (10000-2) for direct transfer of heat;
VIII) optionally separating methylene diphenylene diamine from the mixture of methylene diphenylene diamine and polymethylene polyphenylene polyamine obtained in step VII).

8. Process according to Claim 7, in which process stage (β) C) additionally comprises the following:
IX) workup
• of the aqueous phase from step IA) or
• of the aqueous phase from step IV) or
• of the aqueous phase from step VI) or
• of the water- and aniline-containing stream from step VII) or
• of a mixture of two or more of the aforementioned aqueous process products
in a wastewater workup unit (11000) comprising a wastewater collection vessel (11000-1), optionally a wastewater heater (11000-2), a separation vessel (11000-3), optionally an extraction vessel (11000-4) and a wastewater distillation column (11000-5); wherein the heat-consuming operation from step (ii) is selected from the group consisting of:
• the separation of the water- and aniline-containing stream from the organic phase from step VI) in the distillation stage (10000-1),
• the further purification of the process product remaining in the first distillation stage (10000-1) after removal of the water- and aniline-containing stream in the distillation stage (10000-2), where, in the case of use of stripping vapor for direct heat transfer in (10000-2), this stripping vapor comprises the vapor generated in step (i) and preferably consists solely of the vapor generated in step (i) ,
• if the wastewater heater (11000-2) is present, the heating of this wastewater heater (11000-2), and
• combinations of two or more of the aforementioned operations.

9. Process according to Claim 7 or 8, which additionally comprises the process stage of preparing hydrochloric acid as a further chemical product, wherein the hydrochloric acid is prepared by absorption of hydrogen chloride in water or 0.1% to 20% hydrochloric acid in an absorption column, wherein the hydrochloric acid thus prepared is used as acid in step (β) C) IIA) or (β) C) IB),
wherein the heat-consuming operation is the direct or indirect heating of the bottom of the absorption column from the process stage for preparation of hydrochloric acid.

10. Process according to Claim 3, in which the phosgenation in process stage (α) C) and/or in process stage (β) D) comprises the following:
0) providing gaseous phosgene (3) by evaporating liquid phosgene;
I) evaporating the amine (2) to be phosgenated, reacting the evaporated amine with gaseous phosgene (3) from step 0) in the gas phase, stopping the reaction by contacting with a solvent-containing stream at a temperature below the boiling point of the amine and separating the process product obtained into a liquid stream (60) comprising the crude isocyanate and solvent and a gaseous stream (70) comprising phosgene and hydrogen chloride;
II) separating the liquid stream (60) from step I) into a liquid stream (80) comprising solvent and crude isocyanate, and a gaseous stream (90) comprising phosgene and hydrogen chloride in a distillation apparatus (2100);
III) separating the liquid stream (80) into a gaseous stream (110) comprising solvent and a liquid stream (100) comprising crude isocyanate in a distillation apparatus (2200);
IV) separating the gaseous stream (110), after it has been at least partly liquefied in a condenser (2310), into a liquid stream (120) comprising solvent and a gaseous stream (130) comprising phosgene in a distillation apparatus (2300) ;
V) obtaining a liquid isocyanate stream (140) from the liquid stream (100), giving a gaseous stream (150) comprising secondary components with or without solvents and a mixture of polymeric components and monomeric isocyanate as stream (143), in a distillation apparatus (2400) preferably designed as a dividing wall column.

11. Process according to Claim 10, comprising process chain (α), in which the heat-consuming operation from step (ii) is at least one partial step of the evaporation of liquid phosgene in step 0) and in which the heat-releasing operation from step (i) originates from process stage (α) B).

12. Process according to Claim 3, comprising process chain (α) and process chain (β), in which the at least one heat-releasing operation from step (i) is part of process chain (α) and the at least one heat-consuming operation from step (ii) is part of process chain (β)
or in which
the at least one heat-releasing operation from step (i) is part of process chain (β) and the at least one heat-consuming operation from step (ii) is part of process chain (α).

13. Process according to any of the preceding claims, in which
• in step (i) the vapor is generated by means of an apparatus for indirect heat transfer which is preferably selected from the group consisting of natural circulation boilers, forced circulation boilers, forced passage boilers, shell and tube heat exchangers, U-tube heat exchangers, double tube coil heat exchangers, plate heat exchangers, tube heat exchangers, spiral heat exchangers and inserted condensers or
• in step (ii) the amount of heat required for the performance of the heat-consuming operation is provided by means of an apparatus for indirect heat transfer which is preferably selected from the group consisting of natural circulation evaporators, forced circulation evaporators, inserted evaporators, self-circulation evaporators, rising-film evaporators, falling-film evaporators, plate evaporators, helical tube evaporators, thin-film evaporators, short-path evaporators, shell and tube heat exchangers and spiral heat exchangers or
• in step (i) the vapor is generated by means of an apparatus for indirect heat transfer which is preferably selected from the group consisting of natural circulation boilers, forced circulation boilers, forced passage boilers, shell and tube heat exchangers, U-tube heat exchangers, double tube coil heat exchangers, plate heat exchangers, tube heat exchangers, spiral heat exchangers and inserted condensers, and in step (ii) the amount of heat required for the performance of the heat-consuming operation is provided by means of an apparatus for indirect heat transfer which is preferably selected from the group consisting of natural circulation evaporators, forced circulation evaporators, inserted evaporators, self-circulation evaporators, rising-film evaporators, falling-film evaporators, plate evaporators, helical tube evaporators, thin-film evaporators, short-path evaporators, shell and tube heat exchangers and spiral heat exchangers.

14. Process according to any of the preceding claims, in which, by adjusting
• the size of the vapor stream guided from the vapor-releasing point in step (i) to the position of the heat-consuming operation in step (ii) or
• the nominal width of the pipeline used in step (ii) or
• the size of the vapor stream guided from the vapor-releasing point in step (i) to the position of the heat-consuming operation in step (ii) and the nominal width of the pipeline from step (iii),
a velocity of the vapor stream in the pipeline, calculated from the quotient of the volume flow rate of the vapor and the cross section of the pipeline, in the range from 10.0 m/s to 40.0 m/s is brought about.

15. Process according to any of the preceding claims, in which the pipeline has a length of not more than 400 m and an internal diameter dᵢ in the range from 300.0 mm to 1000 mm.

## Revendications

1. Procédé pour la préparation de *m* isocyanates dans *m* chaînes de procédé, dans lesquelles à chaque fois un isocyanate est préparé en tant que produit final par l'intermédiaire d'au moins un produit intermédiaire,
chacune des *m* chaînes de procédé étant mise en œuvre dans au moins 2 installations de production (100 000-1, 100 000-2,...), à savoir dans une première installation de production (100 000-1) conçue pour la production de l'au moins un produit intermédiaire et dans une deuxième installation de production (100 000-2) conçue pour la préparation du produit final de type isocyanate, les au moins 2 installations de production (100 000-1, 100 000-2,...) faisant partie d'un site de production intégré (1 000 000),
dans lequel dans le site de production intégré (1 000 000) au total n produits chimiques sont produits, qui sont choisis dans le groupe constitué par des produits intermédiaires, des catalyseurs et des produits finaux de type isocyanate, n étant un nombre naturel dans la plage de 2 à 40, préférablement dans la plage de 2 à 20, particulièrement préférablement dans la plage de 2 à 15, et *m* étant un nombre naturel dans la plage de 1 à *n,*
dans lequel, lors de la préparation de chacun des *n* produits chimiques, au moins un processus dégageant de la chaleur ou consommant de la chaleur ayant lieu et dans le procédé pour la préparation des *m* isocyanates au total au moins un processus dégageant de la chaleur et au moins un processus consommant de la chaleur ayant lieu,
(i) l'énergie calorifique libérée dans un processus dégageant de la chaleur de la préparation d'un des produits chimiques dans une première installation de production (100 000-1) étant utilisée au moins en partie pour la génération de vapeur, en particulier de vapeur d'eau, d'une pression de 1,31 bar_{(abs.)} à 1,91 bar_{(abs.)} et d'une température de 107 °C à 119 °C ;
(ii) la vapeur générée en (i) étant utilisée pour la mise en œuvre d'un processus consommant de la chaleur dans la préparation d'un produit chimique différent du produit chimique préparé dans l'étape (i), la préparation de ce produit chimique différent du produit chimique préparé dans l'étape (i) ayant lieu dans une deuxième installation de production (100 000-2), la vapeur générée en (i) étant conduite de l'endroit dégageant de la vapeur dans l'étape (i) par l'intermédiaire d'une conduite vers la position du processus consommant de la chaleur dans l'étape (ii).

2. Procédé selon la revendication 1, dans lequel le produit final de type isocyanate est choisi dans le groupe constitué par un diisocyanate de diphénylméthylène, un polyisocyanate de polyphénylpolyméthylène, des mélanges de diisocyanate de diphénylméthylène et de polyisocyanate de polyphénylpolyméthylène, un diisocyanate de toluylène, un diisocyanate de xylylène, le diisocyanate de 1,5-pentane, le diisocyanate d'hexamethylène, le diisocyanate d'isophorone et un diisocyanate de naphtyle.

3. Procédé selon la revendication 1 ou 2, dans lequel le produit final de type isocyanate est un isocyanate aromatique et dans lequel dans une première chaîne de procédé (α)
A) dans une première étape de procédé, un hydrocarbure aromatique, en particulier le toluène, est nitré pour donner un composé nitroaromatique, en particulier le dinitrotoluène ;
B) le composé nitro aromatique, en particulier le dinitrotoluène, dans une deuxième étape de procédé, est hydrogéné par hydrogénation pour donner une amine aromatique, en particulier la toluylènediamine ;
C) l'amine aromatique, en particulier la toluylènediamine, dans une troisième étape de procédé, est transformée par phosgénation en produit final de type isocyanate aromatique, en particulier en diisocyanate de toluylène ;
et/ou
dans une deuxième chaîne de procédé (β)
A) dans une première étape de procédé, un hydrocarbure aromatique, en particulier le benzène, est nitré pour donner un composé nitroaromatique, en particulier le nitrobenzène ;
B) le composé nitro aromatique, en particulier le nitrobenzène, dans une deuxième étape de procédé, est hydrogéné par hydrogénation pour donner une amine aromatique, en particulier l'aniline ;
C) l'amine aromatique, en particulier l'aniline, dans une troisième étape de procédé, est transformée en une autre amine aromatique, en particulier un diaminodiphénylméthylène, un polyaminopolyphénylpolyméthylène et/ou des mélanges de diaminodiphénylméthylène et de polyaminopolyphénylpolyméthylène ;
D) l'amine aromatique, en particulier un diaminodiphénylméthylène, un polyaminopolyphénylpolyméthylène et/ou des mélanges de diaminodiphénylméthylène et de polyaminopolyphénylpolyméthylène, dans une quatrième étape de procédé, est transformée par phosgénation en produit final de type isocyanate aromatique, en particulier en diisocyanate de diphénylméthylène, en polyisocyanate de polyphénylpolyméthylène et/ou en mélanges de diisocyanate de diphénylméthylène et de polyisocyanate de polyphénylpolyméthylène.

4. Procédé selon la revendication 3, dans lequel la phosgénation selon l'étape de procédé (α) C) et/ou selon l'étape de procédé (β) D) comprend ce qui suit
I) transformation de l'amine (2) devant être traitée au phosgène avec du phosgène (3) en phase liquide et séparation du produit de procédé obtenu en un flux liquide (60) contenant l'isocyanate brut et du solvant et en un flux gazeux (70) contenant du phosgène et du chlorure d'hydrogène ;
II) séparation du flux liquide (60) de l'étape I) en un flux liquide (80) contenant du solvant et l'isocyanate brut et en un flux gazeux (90) contenant du phosgène et du chlorure d'hydrogène dans un dispositif de distillation (2100);
III) séparation du flux liquide (80) en un flux gazeux (110) contenant du solvant et en un flux liquide (100) contenant l'isocyanate brut dans un dispositif de distillation (2200) ;
IV) séparation du flux gazeux (110) après sa liquéfaction au moins partielle dans un condensateur (2310), en un flux liquide (120) contenant du solvant et en un flux gazeux (130) contenant du phosgène dans un dispositif de distillation (2300) ;
V) obtention d'un flux liquide (140) d'isocyanate à partir du flux liquide (100), un flux gazeux (150) contenant un composant secondaire et éventuellement du solvant étant produit, dans un dispositif de distillation (2400), éventuellement comprenant une étape V.1), la séparation de fractions polymériques d'isocyanate dans un dispositif en aval pour la séparation de polymère (2410) en tant que flux (141) .

5. Procédé selon la revendication 4, comprenant la chaîne de procédé (β), l'amine devant être traitée au phosgène dans l'étape de procédé (β) D) étant un mélange de diaminodiphénylméthylène et de polyaminopolyphénylpolyméthylène, l'étape V.1) étant en outre mise en œuvre et le produit final de type isocyanate étant produit dans les deux flux 140 et 141, le flux 140 comprenant du diisocyanate de diphénylméthylène et le flux 141 comprenant un mélange de diisocyanate de diphénylméthylène et de polyisocyanate de polyphénylpolyméthylène.

6. Procédé selon la revendication 5, dans lequel le processus dégageant de la chaleur de l'étape (i) est la liquéfaction au moins partielle du flux gazeux (110) dans le condensateur (2310) et dans lequel la vapeur générée par le dégagement de chaleur est conduite dans l'étape de procédé (β) C).

7. Procédé selon la revendication 6, dans lequel l'étape de procédé (β) C) comprend les étapes suivantes :
IA) réaction d'aniline et de formaldéhyde dans un réacteur (3000) en absence d'un catalyseur acide avec formation d'un aminal, suivie par la séparation du mélange réactionnel obtenu en une phase aqueuse et une phase organique contenant l'aminal dans un dispositif de séparation de phases intégré dans le réacteur ou dans un appareil de séparation de phases distinct (4000) ;
IIA) réaction d'au moins une partie de la phase organique contenant l'aminal obtenue dans l'étape IA) dans un réacteur (5000) avec un acide, l'aminal réagissant pour donner un mélange de diaminodiphénylméthylène et de polyaminopolyphénylpolyméthylène ;
ou
IB) réaction d'aniline et d'acide dans un réacteur ;
IIB) réaction d'au moins une partie du mélange réactionnel obtenu dans l'étape IB) dans un réacteur avec du formaldéhyde pour donner un mélange de diaminodiphénylméthylène et de polyaminopolyphénylpolyméthylène ;
III) neutralisation du mélange réactionnel obtenu dans l'étape IIA) ou IIB) dans un réacteur (6000) ;
IV) séparation du mélange réactionnel neutralisé obtenu dans l'étape III) en une phase organique, comprenant un mélange de diaminodiphénylméthylène et de polyaminopolyphénylpolyméthylène, et en une phase aqueuse dans un récipient de séparation (7000) ;
V) lavage de la phase organique avec un liquide de lavage dans un récipient de lavage (8000) ;
VI) séparation du mélange obtenu dans l'étape V) en une phase organique, comprenant un mélange de diaminodiphénylméthylène et de polyaminopolyphénylpolyméthylène, et en une phase aqueuse dans un récipient de séparation (7000) ;
VII) distillation de la phase organique de l'étape VI) dans un appareil de distillation (10000) comprenant un premier étage de distillation (10000-1) pour la séparation d'un flux contenant de l'eau et de l'aniline et un deuxième étage de distillation (10000-2) pour la purification supplémentaire du produit de procédé restant dans la première étape après séparation du flux contenant de l'eau et de l'aniline avec obtention d'un mélange de diaminodiphénylméthylène et de polyaminopolyphénylpolyméthylène, dans lequel, dans la deuxième étape (10000-2), éventuellement de la vapeur de strippage est utilisée pour un transfert direct de chaleur ;
VIII) séparation éventuelle de diaminodiphénylméthylène du mélange de diaminodiphénylméthylène et de polyaminopolyphénylpolyméthylène obtenu dans l'étape VII).

8. Procédé selon la revendication 7, dans lequel l'étape de procédé (β) C) comprend de plus ce qui suit :
IX) traitement
• de la phase aqueuse de l'étape IA) ou
• de la phase aqueuse de l'étape IV) ou
• de la phase aqueuse de l'étape VI) ou
• du flux contenant de l'eau et de l'aniline de l'étape VII) ou
• d'un mélange de deux ou plus des produits de procédé aqueux mentionnés précédemment
dans un dispositif de traitement d'eaux résiduaires (11000) comprenant un récipient de collecte d'eaux résiduaires (11000-1), éventuellement un réchauffeur d'eaux résiduaires (11000-2), un récipient de séparation (11000-3), éventuellement un récipient d'extraction (11000-4) et une colonne de distillation d'eaux résiduaires (11000-5) ;
le processus consommant de la chaleur de l'étape (ii) étant choisi dans le groupe constitué par :
• la séparation du flux contenant de l'eau et de l'aniline de la phase organique de l'étape VI) dans un étage de distillation (10000-1),
• la purification supplémentaire du produit de procédé restant dans le premier étage de distillation (10000-1) après séparation du flux contenant de l'eau et de l'aniline dans l'étage de distillation (10000-2), dans lequel, lors de l'utilisation de vapeur de strippage pour un transfert direct de chaleur dans (10000-2), cette vapeur de strippage comprend la vapeur générée dans l'étape (i) et préférablement n'est constituée que de la vapeur générée dans l'étape (i),
• pour autant que le réchauffeur d'eaux résiduaires (11000-2) est présent, le réchauffage de ce réchauffeur d'eaux résiduaires (11000-2) et
• une combinaison de deux ou plus des processus mentionnés précédemment.

9. Procédé selon la revendication 7 ou 8, qui comprend de plus l'étape de procédé de préparation d'acide chlorhydrique comme produit chimique supplémentaire, l'acide chlorhydrique étant préparé par absorption de chlorure d'hydrogène dans de l'eau ou d'acide chlorhydrique d'une concentration de 0,1 % à 20 % dans une colonne d'absorption, l'acide chlorhydrique ainsi préparé étant utilisé comme acide dans l'étape (β) C) IIA) ou (β) C) IB),
le processus consommant de la chaleur étant le chauffage direct ou indirect du fond de la colonne d'absorption de l'étape de procédé de la préparation d'acide chlorhydrique.

10. Procédé selon la revendication 3, dans lequel la phosgénation dans l'étape de procédé (α) C) et/ou dans l'étape de procédé (β) D) comprend ce qui suit
0) mise à disposition de phosgène gazeux (3) par vaporisation de phosgène liquide ;
I) vaporisation de l'amine (2) devant être traitée au phosgène, transformation de l'amine vaporisée avec du phosgène gazeux (3) de l'étape 0) en phase gazeuse, arrêt de la transformation par mise en contact avec un flux contenant un solvant d'une température inférieure au point d'ébullition de l'amine et séparation du produit de procédé obtenu en un flux liquide (60) contenant l'isocyanate brut et du solvant et en un flux gazeux (70) contenant du phosgène et du chlorure d'hydrogène ;
II) séparation du flux liquide (60) de l'étape I) en un flux liquide (80) contenant du solvant et de l'isocyanate brut et en un flux gazeux (90) contenant du phosgène et du chlorure d'hydrogène dans un dispositif de distillation (2100) ;
III) séparation du flux liquide (80) en un flux gazeux (110) contenant du solvant et en un flux liquide (100) contenant l'isocyanate brut dans un dispositif de distillation (2200) ;
IV) séparation du flux gazeux (110) après sa liquéfaction au moins partielle dans un condensateur (2310), en un flux liquide (120) contenant du solvant et en un flux gazeux (130) contenant du phosgène dans un dispositif de distillation (2300) ;
V) obtention d'un flux liquide (140) d'isocyanate à partir du flux liquide (100), un flux gazeux (150) contenant un composant secondaire et éventuellement du solvant et un mélange de parties polymériques et d'isocyanate monomérique en tant que flux (143) étant produits, dans un dispositif de distillation (2400) préférablement conçu comme colonne à paroi de séparation.

11. Procédé selon la revendication 10, comprenant la chaîne de procédé (α), dans lequel le processus consommant de la chaleur de l'étape (ii) est au moins une étape partielle de la vaporisation du phosgène liquide dans l'étape 0) et dans lequel le processus dégageant de la chaleur de l'étape (i) provient de l'étape de procédé (α) B).

12. Procédé selon la revendication 3, comprenant la chaîne de procédé (α) et la chaîne de procédé (β), dans lequel
l'au moins un processus dégageant de la chaleur de l'étape (i) fait partie de la chaîne de procédé (α) et l'au moins un processus consommant de la chaleur de l'étape (ii) fait partie de la chaîne de procédé (β)
ou dans lequel
l'au moins un processus dégageant de la chaleur de l'étape (i) fait partie de la chaîne de procédé (β) et l'au moins un processus consommant de la chaleur de l'étape (ii) fait partie de la chaîne de procédé (α).

13. Procédé selon l'une quelconque des revendications précédentes, dans lequel
• dans l'étape (i), la vapeur est générée au moyen d'un appareil pour un transfert indirect de chaleur, lequel est préférablement choisi dans le groupe constitué par des chaudières à circulation naturelle, des chaudières à circulation forcée, des chaudières à flux forcé, des échangeurs de chaleur à faisceau de tubes, des échangeurs de chaleur à tube en U, des échangeurs de chaleur à serpentin à double tube, des échangeurs de chaleur à plaques, des échangeurs de chaleur à tubes, des échangeurs de chaleur en spirale et des condensateurs enfichables ou
• dans l'étape (ii) la quantité de chaleur nécessaire pour la mise en œuvre du processus consommant de la chaleur est fournie par un appareil pour un transfert indirect de chaleur, lequel est préférablement choisi dans le groupe constitué par des évaporateurs à circulation naturelle, des évaporateurs à circulation forcée, des évaporateurs enfichables, des évaporateurs à circuit fermé, des évaporateurs grimpants, des évaporateurs à film tombant, des évaporateurs à plaques, des évaporateurs à tube hélicoïdal, d'évaporateurs en couche mince, des évaporateurs d'évaporation rapide, des échangeurs de chaleur à faisceau de tubes et des échangeurs de chaleur en spirale ou
• dans l'étape (i), la vapeur est générée au moyen d'un appareil pour un transfert indirect de chaleur, lequel est préférablement choisi dans le groupe constitué par des chaudières à circulation naturelle, des chaudières à circulation forcée, des chaudières à flux forcé, des échangeurs de chaleur à faisceau de tubes, des échangeurs de chaleur à tube en U, des échangeurs de chaleur à serpentin à double tube, des échangeurs de chaleur à plaques, des échangeurs de chaleur à tubes, des échangeurs de chaleur en spirale et des condensateurs enfichables et dans l'étape (ii), la quantité de chaleur nécessaire pour la mise en œuvre du processus consommant de la chaleur est fournie par un appareil pour un transfert indirect de chaleur, lequel est préférablement choisi dans le groupe constitué par des évaporateurs à circulation naturelle, des évaporateurs à circulation forcée, des évaporateurs enfichables, des évaporateurs à circuit fermé, des évaporateurs grimpants, des évaporateurs à film tombant, des évaporateurs à plaques, des évaporateurs à tube hélicoïdal, d'évaporateurs en couche mince, des évaporateurs d'évaporation rapide, des échangeurs de chaleur à faisceau de tubes et des échangeurs de chaleur en spirale.

14. Procédé selon l'une quelconque des revendication précédentes, dans lequel par l'ajustement
• de la taille du flux de vapeur conduit de l'endroit dégageant de la vapeur dans l'étape (i) à la position du processus consommant de la chaleur dans l'étape (ii) ou
• de la taille nominale de la conduite utilisée dans l'étape (ii) ou
• de la taille du flux de vapeur conduit de l'endroit dégageant de la vapeur dans l'étape (i) à la position du processus consommant de la chaleur dans l'étape (ii) et de la taille nominale de la conduite de l'étape (iii),
une vitesse du flux de vapeur dans la conduite, calculée à partir du quotient du débit volumique de la vapeur et de la section de la conduite, dans la plage de 10,0 m/s à 40,0 m/s, est réalisée.

15. Procédé selon l'une quelconque des revendications précédentes, dans lequel la conduite présente une longueur maximale de 400 m et un diamètre intérieur dᵢ dans la plage de 300,0 mm à 1 000 mm.
